(19) Europäisches Patentamt
European Patent Office
Office européen des brevets

(11) EP 0 962 812 A1

(12) **EUROPEAN PATENT APPLICATION**

(43) Date of publication:
**08.12.1999 Bulletin 1999/49**

(51) Int. Cl.[6]: **G03C 1/498**, C07C 51/41

(21) Application number: **99110902.6**

(22) Date of filing: **02.06.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **03.06.1998 JP 17063898**
**18.06.1998 JP 18821098**

(71) Applicant:
**FUJI PHOTO FILM CO., LTD.**
**Kanagawa 250-01 (JP)**

(72) Inventors:
- **Oyamada, Takayoshi**
  **Minami-Ashigara-shi, Kanagawa-ken 258 (JP)**
- **Ohzeki, Tomoyuki**
  **Minami-Ashigara-shi, Kanagawa-ken 258 (JP)**

(74) Representative: **HOFFMANN - EITLE**
**Patent- und Rechtsanwälte**
**Arabellastrasse 4**
**81925 München (DE)**

# (54) Aqueous dispersion of fatty acid silver salt particles, method for redispersing fatty acid silver salt particles, photothermographic light-sensitive material, and method for producing the same

(57) Fatty acid silver salt particles of an aqueous dispersion thereof which are formed by simultaneously adding into a reaction vessel (a) a silver ion-containing aqueous solution or a silver ion-containing water-organic solvent mixed solution and (b) an aqueous solution of an alkali metal salt of a fatty acid, a solution thereof in a water-organic solvent mixed solvent or a solution thereof in an organic solvent, in an amount of at least 10% of the total amount of silver to be added, and the fatty acid silver salt particles formed satisfies all the following characteristics:

(1) The average equivalent-sphere diameter of the fatty acid silver salt particles is from 0.1 μm to 0.8 μm;
(2) The average ratio of long sides/short sides in main planes of the fatty acid silver salt particles is from 1 to 4;
(3) The average aspect ratio of the particle is from 2 to 30; and
(4) The average thickness of the particles is from 0.01 μm to 0.20 μm.

EP 0 962 812 A1

## Description

FIELD OF THE INVENTION

The present invention relates to fatty acid silver salt particles used in a photothermographic light-sensitive material, a method for producing the same, a method for redispersing the fatty acid silver salt particles, a photothermographic light-sensitive material using the fatty acid silver salt, and a method for producing the same.

BACKGROUND OF THE INVENTION

In the recent medical field, it has been eagerly desired to reduce the amount of processing waste liquor, from the viewpoints of environmental preservation and space saving. Accordingly, techniques relating to photothermographic light-sensitive materials for medical diagnosis and photomechanical processes have been required which can be efficiently exposed with a laser image setter or a laser imager and can form sharp black images having high resolution. These photothermographic light-sensitive materials can be provided without the use of processing chemicals of the solution, so that they can provide to customers heat development systems which are simpler and do not damage the environment.

Methods for forming images by heat development are described, for example, in U.S. Patents 3,152,904 and 3,457,075, and D. Morgan and B. Shely, Thermally Processed Silver Systems (Image Processes and Materials), Neblette, the eighth edition, edited by Sturge, V. Walworth and A. Shepp, page 2 (1969).

Such photothermographic light-sensitive materials contain reducible light-insensitive silver sources (for example, organic silver salts), catalytic active amounts of photocatalysts (for example, silver halides) and reducing agents for silver usually in the state where they are dispersed in organic binder matrixes. The light-sensitive materials are stable at room temperature. However, when they are heated at a high temperature (for example, 80°C or higher) after exposure, silver is produced by the oxidation-reduction reaction of the reducible silver sources (which functions as oxidizing agents) with the reducing agents. The oxidation-reduction reaction is accelerated by the catalytic action of latent images generated by exposure. Silver produced by the reaction of the reducible silver salts in exposed areas provides black images, which make a contrast with unexposed areas to form images.

The silver sources used in such a system are generally silver salts of fatty acids, and various manufacturing methods have been known. For example, there are a method of preparing organic silver salts in coexisting solutions of water and solvents sparingly soluble in water, as described in JP-A-49-93310 (the term "JP-A" as used herein means an "unexamined published Japanese patent application"), JP-A-49-94619 and JP-A-53-68702, a method of preparing organic silver salts in aqueous solutions, as described in JP-A-53-31611, JP-A-54-4117 and JP-A-54-46709, and a method of preparing organic silver salts in organic solvents, as described in JP-A-57-186745, JP-A-47-9432 and U.S. Patent 3,700,458. Basically, the silver salts of the fatty acids are each prepared by melting the fatty acid by heating in water at a melting point thereof or higher, and adding sodium hydroxide or an alkali metal salt thereto with vigorous stirring, followed by addition of silver nitrate for converting alkali soap to silver soap.

Such alkali soap forms micelles in aqueous solutions to provide apparently clouded solutions. The reaction of from the alkali soap in such a micellar state to the silver soap frequently brings about a problem in respect to production stability. Accordingly, as a method for preparing homogeneous solutions of alkali soap, the use of mixed solutions of solvents, water and alcohols is disclosed in JP-A-55-40607.

Further, the alkali soap exhibits alkalinity as shown by the name thereof. In this case, therefore, the silver soap is produced at a high pH. However, addition of silver nitrate to alkali solutions causes formation of silver oxide as a by-product. Further, slight pollution with reducing materials inevitable in the production and not intended brings about high reducibility because of the high pH, which causes formation of silver nuclei not intended. The formation of such a by-product is very disadvantageous in the performances of the photothermographic light-sensitive materials, particularly, in that undesirable fog is developed. From the above-mentioned viewpoint, the problem of fog is not solved yet also by the method described in JP-A-55-40607.

Further, JP-A-9-127643 discloses a method for forming silver salts by simultaneous metering addition of alkali metal salt solutions and silver nitrate solutions, and describes simultaneous addition of a solution of sodium behenate in a mixed solvent of water and isopropyl alcohol and a silver nitrate solution. Silver behenate formed by this method can lower the reaction at a high pH to a neutral region, thereby decreasing the amount of silver oxide formed. Then, silver behenate particles formed by this method had a diameter of 0.04 μm to 0.06 μm according to a transmission electron photomicrograph, had no clear selective growing direction, and were fine particles apparently not needle-like. However, the silver behenate particles formed by this method was too fine to allow the keeping quality to be compatible with the light transmission properties.

Further, isopropyl alcohol is also reductive. In this respect, therefore, the problem of fog is not completely solved.

Care should be variously taken for the preparation of the fatty acid silver salts, and it is necessary to exclude reducing materials as much as possible, to control the particle size, and further to control the particle shape, in forming the fatty acid silver salts.

Furthermore, the temperature of a reaction solution in a reaction vessel is closely related to inhibition of the reducing reaction, and the particle size, size distribution and shape of the fatty acid silver salt to be prepared, and it is preferred that the temperature is low. However, the conventional information has not given no suggestion therefor.

In addition, the concentration of silver behenate in forming the silver behenate particles has been mainly 0.10 mol/liter or less, and an increase in the concentration of silver behenate in the particle formation has been an important problem for cost reduction.

Then, many of the photothermographic light-sensitive materials in which the fatty acid silver salts are used have hitherto been applied in the form of coating solutions in which organic solvents such as toluene, methyl ethyl ketone (MEK) and methanol are used as solvents, thereby forming emulsion layers. The use of organic solvents as solvents is very disadvantageous in terms of safety and adverse effects to the human body in manufacturing processes, and cost for the recovery of the solvents and the like.

Accordingly, methods for forming light-sensitive layers by use of coating solutions of aqueous solvents have been proposed. For example, the use of gelatin as a binder is described in JP-A-49-52626 and JP-A-53-116144. Further, the use of polyvinyl alcohol as a binder is described in JP-A-50-151138.

Furthermore, an example in which gelatin is used in combination with polyvinyl alcohol is described in JP-A-60-61747. As another example, the use of water-soluble polyvinyl acetal as a binder is described in JP-A-58-28737.

Surely, the use of such binders permits the formation of light-sensitive layers using coating solutions of aqueous solvents, resulting in high environmental and cost merit.

However, when the above-mentioned polymers are used as binders, the compatibility with the fatty acid silver salts is poor, which causes a failure to obtain coated products fit for practical applications in respect to the quality of coated surfaces, and a phenomenon occurs that the color tone of silver in developed areas turns brown or yellow, far from black, which is considered to be inherently preferred, or that fog is highly developed, resulting in significant deterioration of commodity value.

For solving such a problem, EP-A-803764A1 discloses photothermographic light-sensitive materials in which thermoplastic resins or polymers having a equilibrium moisture content of 2% by weight or less at 25°C at 60% RH are dispersed in aqueous solvents, and used as binders.

It has been found that for conducting such aqueous solvent coating, the fatty acid silver salts are required to be also dispersed in the coating solutions of the aqueous solvents, and that for obtaining coated products fit for practical applications in respect to the quality of coated surfaces by use of the aqueous solvent coating solutions containing the fatty acid silver salts, a state is required in which the fatty acid silver salts are finely dispersed in aqueous solvents without coagulation.

The fine particles of the fatty acid silver salts can be mechanically dispersed by known means for finely dispersing particles (for example, high-speed mixers, homogenizers, high-speed impact ball mills, Banbury mixers, homomixers, kneaders, ball mills, vibrating ball mills, planetary ball mills, attriters, sand mills, bead mills, colloid mills, jet mills, roller mills, trommel and highspeed stone mills) in the presence of supplementary dispersing agents. According to these methods, however, particle coagulation much occurs. As a result, only coating solutions poor in the quality of coated surfaces are obtained.

From the above, no means for finely dispersing the particles of the fatty acid silver salts for the aqueous solvent coating solutions of the fatty acid silver salts good in the quality of coated surfaces has been discovered.

## SUMMARY OF THE INVENTION

An object of the present invention is to provide a method for preparing fatty acid silver salt particles excellent in antifogging ability when used in an aqueous solvent, antifogging ability with time, image stability after heat development, and light transmission properties.

Another object of the present invention is to provide a method for producing a fatty acid silver salt improved in the quality of a coated surface.

A further object of the present invention is to provide a method for producing a photothermographic light-sensitive material excellent in these performances using such a fatty acid silver salt.

These objects have been attained by the following means:

(1) An aqueous dispersion (i.e., a water-based dispersion) of fatty acid silver salt particles which are dispersed in an aqueous solvent, wherein the fatty acid silver salt particles are ones formed by simultaneously adding into a reaction vessel (a) a silver ion-containing aqueous solution or a silver ion-containing water-organic solvent mixed solution and (b) an aqueous solution of an alkali metal salt of a fatty acid, a solution thereof in a water-organic solvent mixed solvent or a solution thereof in an organic solvent, in an amount of at least 10% of the total amount of silver to be added, and the fatty acid silver salt particles formed satisfies all the following characteristics:

(1) The average equivalent-sphere diameter of the fatty acid silver salt particles is from 0.1 μm to 0.8 μm;
(2) The average ratio of long sides/short sides in main planes of the fatty acid silver salt particles is from 1 to 4;
(3) The average aspect ratio of the particle is from 2 to 30; and
(4) The average thickness of the particles is from 0.01 μm to 0.20 μm;

(2) The aqueous dispersion of the fatty acid silver salt particles described in the above (1), wherein the fatty acid silver salt particle formed by simultaneously adding the components (a) and (b) at least 50% of the total amount of silver to be added;
(3) The aqueous dispersion of the fatty acid silver salt particles described in the above (1) or (2), wherein the coefficient of variation of the size of the fatty acid silver salt particles formed is 20% or less;
(4) The aqueous dispersion of the fatty acid silver salt particles described in any one of the above (1) to (3), wherein the maximum concentration of the fatty acid silver salt particles during particle formation is 0.12 mol/liter to 0.5 mol/liter;
(5) A method for producing an aqueous dispersion of fatty acid silver salt particles which are dispersed in an aqueous solvent, which comprises simultaneously adding into a reaction vessel (a) a silver ion-containing aqueous solution or a silver ion-containing water-organic solvent mixed solution and (b) an aqueous solution of an alkali metal salt of a fatty acid, a solution thereof in a water-organic solvent mixed solvent or a solution thereof in an organic solvent, in an amount of at least 10% of the total amount of silver to be added, the fatty acid silver salt particles formed satisfying all the following characteristics:

(1) The average equivalent-sphere diameter of the fatty acid silver salt particles is from 0.1 μm to 0.8 μm;
(2) The average ratio of long sides/short sides in main planes of the fatty acid silver salt particles is from 1 to 4;
(3) The average aspect ratio of the particle is from 2 to 30; and
(4) The average thickness of the particles is from 0.01 μm to 0.20 μm;

(6) The method described in the above (5), wherein at least 50% of the total amount of silver to be added of the components (a) and (b) is simultaneously added;
(7) The method described in the above (5) or (6), wherein the coefficient of variation of the size of the fatty acid silver salt particles formed is 20% or less;
(8) The method described in any one of the above (5) to (7), wherein the maximum concentration of the fatty acid silver salt particles during particle formation is 0.12 mol/liter to 0.5 mol/liter;
(9) The method described in the above (5), wherein an aqueous solution of a tertiary alcohol containing the fatty acid alkali metal salt is added to the solution in the reaction vessel, and the temperature difference between the solution in the reaction vessel and the aqueous solution of the tertiary alcohol containing the fatty acid alkali metal salt is from 30°C to 85°C;
(10) The method described in the above (9), wherein a tip of a nozzle for adding the aqueous solution of the tertiary alcohol containing said fatty acid alkali metal salt to the solution in the reaction vessel does not come into contact with the solution in said reaction vessel;
(11) The method described in the above (9) or (10), wherein a path of from a vessel in which the aqueous solution of the tertiary alcohol containing said fatty acid alkali metal salt is prepared to the nozzle for adding the aqueous solution to the solution in the reaction vessel is kept hot so as to maintain the aqueous solution of the tertiary alcohol containing the fatty acid alkali metal salt in the transfer path at a constant temperature of 65°C to 85°C;
(12) The method described in any one of the above (5) to (11), which further comprises a desalting process after formation of the fatty acid silver salt;
(13) A method for redispersing the fatty acid silver salt particles of the aqueous dispersion described in any one of the above (1) to (4) in an aqueous solvent, which comprises crudely dispersing the fatty acid silver salt particles in the aqueous solvent to prepare a crude dispersion, and then, converting the crude dispersion to a high-pressure high-speed stream, followed by a reduction in pressure;
(14) A photothermographic light-sensitive material comprising a support having thereon a light-sensitive layer containing at least a fatty acid silver salt, a reducing agent for silver ions, a binder and a light-sensitive silver halide, wherein the light-sensitive layer is formed by applying a coating solution in which 30% by weight or more of a solvent is water, and drying it, and the coating solution for the light-sensitive layer contains a polymer dispersed in a latex form as a main binder, and the fatty acid silver salt particles of the aqueous dispersion described in any one of the above (1) to (4) as a dispersion;
(15) A method for producing a photothermographic light-sensitive material by applying a coating solution for a light-sensitive layer containing a fatty acid silver salt, a reducing agent for silver ions, a binder and a light-sensitive silver halide onto a support, and drying it, wherein the coating solution for the light-sensitive layer contains 30% by weight or more of water as a solvent, a polymer dispersed in a latex form as a main binder, and the fatty acid silver salt particles dispersed by the redispersing method described in the above (13) as a dispersion; and

(16) The method described in the above (15), wherein at least one metal ion selected from the group consisting of Ca, Mg, Ce, Al, Zn and Ba is added in any step from a step for producing the fatty acid silver salt to a step for applying the coating solution for the light-sensitive image forming layer containing the fatty acid silver salt obtained by the producing method.

## BRIEF DESCRIPTION OF THE DRAWINGS

Fig. 1 is a schematic view showing one embodiment of an apparatus used for conducting a producing method of the present invention;
Fig. 2 is a schematic view for illustrating positions to which nozzles are attached;
Fig. 3 is a schematic view for illustrating positions to which nozzles are attached; and
Fig. 4 is another schematic view for illustrating positions to which nozzles are attached.

## DETAILED DESCRIPTION OF THE INVENTION

The present invention will be described below in more detail.

The pH of the silver ion-containing aqueous solution or the silver ion-containing water-organic solvent mixed solution used in the present invention is preferably from 1 to 6, and more preferably from 1.5 to 4. Further, for adjustment of the pH, any acids and alkalis can be added.

The silver ion-containing aqueous solution or the silver ion-containing water-organic solvent mixed solution used in the present invention can be adjusted with silver nitrate so as to give a specified silver ion concentration.

The silver ion concentration of the silver ion-containing aqueous solution or the silver ion-containing water-organic solvent mixed solution used in the present invention is arbitrarily determined. However, it is preferably from 0.03 mol/liter to 6.5 mol/liter, and more preferably from 0.1 mol/liter to 5 mol/liter, as the molar concentration.

For forming the fatty acid salt particles of the present invention, at least one of the silver ion solution, the fatty acid alkali metal salt solution and a solution previously prepared in a reaction field should contain an organic solvent in such an amount that the fatty acid alkali metal salt can be dissolved therein to form a substantially transparent solution without formation of string-like associated materials and micelles. Although an organic solvent may be used alone for the solution, a water-organic solvent mixed solution may also be used.

The organic solvents used in the present invention may be any, as long as they are water-miscible and have the above-mentioned properties. However, solvents deteriorating the photographic characteristics are undesirable. The solvents are preferably alcohols and acetone miscible with water, more preferably tertiary alcohols, and still more preferably tertiary alcohols each having 4 to 6 carbon atoms.

In the solution of the alkali metal salt of the fatty acid in the water-organic solvent mixed solvent used in the present invention, the amount of the organic solvent used for obtaining the uniformity of the solution is from 3% to 70%, and preferably from 5% to 50%, as the solvent volume, based on the volume of water. In this case, the optimum solvent volume varies depending on the reaction temperature, so that the optimum amount can be determined by trial and error.

As the aqueous solution of the tertiary alcohol containing the fatty acid alkali metal salt used in the present invention, a mixed solvent of water and a tertiary alcohol having 4 to 6 carbon atoms is preferred for obtaining the uniformity of the solution. If the number of carbon atoms exceeds this range, the compatibility with water is unfavorably lost. Of the tertiary alcohols having 4 to 6 carbon atoms, tert-butanol most compatible with water is most preferred. Alcohols other than the tertiary alcohols are reductive and have disadvantageous effects in forming the fatty acid silver salts, so that they are unfavorable as described above. The amount of the tertiary alcohol which may be used in the aqueous solution of the tertiary alcohol containing the fatty acid alkali metal salt is from 3% to 70%, and preferably from 5% to 50%, as solvent volume based on the volume of water in the aqueous solution of the tertiary alcohol.

The silver salt of the fatty acid used in the present invention is a silver salt which is relatively stable to light, and forms a silver image when heated to a temperature of 80°C or more in the presence of an exposed photocatalyst (such as a latent image of a light-sensitive silver halide) and a reducing agent. The fatty acids are long-chain aliphatic carboxylic acids each having particularly 10 to 30 carbon atoms, and preferably 12 to 26 carbon atoms. Preferred examples of the aliphatic carboxylic acids include cerotic acid, lignoceric acid, behenic acid, erucic acid, arachidic acid, stearic acid, oleic acid, lauric acid, caproic acid, myristic acid, palmitic acid, maleic acid, fumaric acid, tartaric acid, linoleic acid, butyric acid, camphoric acid and mixtures thereof.

The alkali metal of the alkali metal salt of the fatty acid used in the present invention is specifically Na or K. The alkali metal salt of the fatty acid is prepared by adding NaOH or KOH to the fatty acid. At this time, it is preferred that the amount of the alkali is not more than the amount equivalent to the fatty acid to allow the unreacted fatty acid to remain. In this case, the amount of the residual fatty acid is from 3 mol% to 50 mol%, and preferably from 3 mol% to 30 mol%, based on the total fatty acid. Further, the alkali metal salt of the fatty acid may be prepared by adding the alkali in an amount of not less than a desired amount, and thereafter, adding an acid such as nitric acid or sulfuric acid to neutralize the excess alkali.

Further, the pH can be adjusted according to the characteristics necessary for the fatty acid silver salts. For the pH adjustment, any acids and alkalis can be used.

Further, for example, a compound as represented by formula (1) of JP-A-62-65035, a water-soluble group-containing N-heterocyclic compound as described in JP-A-62-150240, an inorganic peroxide as described in JP-A-50-101019, a sulfur compound as described in JP-A-51-78319, a disulfide compound as described in JP-A-51-78319 and hydrogen peroxide can be added to the silver ion-containing aqueous solution or the silver ion-containing water-organic solvent mixed solution and the aqueous solution of the alkali metal salt of the fatty acid, the solution thereof in the water-organic solvent mixed solvent or the solution thereof in the organic solvent, or the solution in the reaction vessel to which both the solutions are added.

The aqueous solution containing the water-soluble silver salt may contain the tertiary alcohol having 4 to 6 carbon atoms. In that case, the volume of the aqueous solution of the water-soluble silver salt is 70% or less, and preferably 50% or less, based on the total volume. Further, the temperature of the aqueous solution is preferably from 0°C to 50°C, and more preferably from 5°C to 30°C. As described later, when the aqueous solution containing the water-soluble silver salt and the aqueous solution of the tertiary alcohol containing the alkali metal salt of the fatty acid are simultaneously added, the temperature is most preferably from 5°C to 15°C.

The concentration of the alkali metal salt of the fatty acid used in the present invention is from 5% by weight to 50% by weight, preferably from 7% by weight to 45% by weight, and more preferably from 10% by weight to 40% by weight.

In the present invention, the silver ion-containing aqueous solution or the silver ion-containing water-organic solvent mixed solution and the aqueous solution of the alkali metal salt of the fatty acid, the solution thereof in the water-organic solvent mixed solvent or the solution thereof in the organic solvent are simultaneously added, thereby preparing a desired fatty acid silver salt. In that case, it is preferred that 10% to 100% of total amount of silver added is added simultaneously with the aqueous solution of the approximately equimolar alkali metal salt of the fatty acid or the solution thereof in the water-organic solvent mixed solvent. It is more preferred that 30% to 100% is simultaneously added, and it is still more preferred that 50% to 100% is simultaneously added. In particular, simultaneous addition of 70% to 100% is yet still more preferred. When either of them is previously added, it is preferred that the Ag ion-containing solution is previously added.

For obtaining the form of the present invention, the silver ion-containing aqueous solution or the silver ion-containing water-organic solvent mixed solution and the aqueous solution of the alkali metal salt of the fatty acid, the solution thereof in the water-organic solvent mixed solvent or the solution thereof in the organic solvent can be adjusted to appropriate temperatures. The temperature of the silver ion-containing solution is preferably from 5°C to 60°C, more preferably from 5°C to 40°C, still more preferably from 5°C to 30°C, and most preferably from 5°C to 15°C, for ensuring the stability of the solution. The temperature of the solution of the alkali metal salt of the fatty acid is preferably from 50°C to 90°C, more preferably from 60°C to 85°C, and most preferably 65°C to 85°C, for keeping the solution at a certain temperature for avoiding the phenomena of crystallization and solidification of the alkali soap.

Further, for constantly controlling the reaction temperature, it is preferred that the reaction temperature is constantly controlled at a certain temperature selected from the above-mentioned range.

The temperature of the reaction solution during silver salt formation may be any. However, it is preferably from 5°C to 70°C, more preferably from 10°C to 50°C, and particularly preferably from 20°C to 45°C, which allows the characteristics as a photographic light-sensitive material to be more improved.

In the present invention, it is preferred that the aqueous dispersion of the fatty acid silver salt particles is prepared by reacting the silver ion-containing aqueous solution (hereinafter also referred to as a water-soluble silver salt-containing aqueous solution) with the aqueous solution of the tertiary alcohol containing the alkali metal salt of the fatty acid in the reaction vessel. In this case, a step of adding the aqueous solution of the tertiary alcohol containing the alkali metal salt of the fatty acid to the solution in the reaction vessel is included. When this aqueous solution of the tertiary alcohol containing the alkali metal salt of the fatty acid is added, the temperature difference between the solution in the reaction vessel (preferably, the water-soluble silver salt-containing aqueous solution previously placed, or when the water-soluble silver salt-containing aqueous solution and the aqueous solution of the tertiary alcohol containing the alkali metal salt of the fatty acid are simultaneously added from the beginning without previously adding the water-soluble silver salt-containing aqueous solution, water or the mixed solvent of water and the tertiary alcohol as described later, and even when the water-soluble silver salt-containing aqueous solution is previously placed, water or the mixed

solvent of water and the tertiary alcohol may be previously placed) and the aqueous solution of the tertiary alcohol containing the alkali metal salt of the fatty acid is preferably from 30°C to 85°C. The crystal form of the fatty acid silver salt is preferably controlled by maintaining such temperature difference during addition of the aqueous solution of the tertiary alcohol containing the alkali metal salt of the fatty acid. When the photothermographic light-sensitive material is prepared, fog (Dmin) is effectively decreased. In contrast, if the temperature difference is decreased to less than 30°C, the controllability of the crystal form of the fatty acid silver salt is deteriorated, and fog is increased. On the other hand, if the temperature difference exceeds 85°C, the production stability can not be maintained.

Further, inclusion of a step of adding the aqueous solution of the tertiary alcohol containing the alkali metal salt of the fatty acid results in a significant reduction in fog.

In the present invention, the temperature difference between the aqueous solution of the tertiary alcohol containing the alkali metal salt of the fatty acid and the solution in the reaction vessel is from 30°C to 85°C, and preferably from 30°C to 75°C. In this case, it is preferred that the temperature of the aqueous solution of the tertiary alcohol containing the alkali metal salt of the fatty acid is higher. This favorably controls the speed at which the high-temperature aqueous solution of the tertiary alcohol containing the alkali metal salt of the fatty acid is precipitated in a fine crystal form by rapid cooling in the reaction vessel and the speed at which the fatty acid silver salt is formed by the reaction with the water-soluble silver salt. Thus, the crystal form of the fatty acid silver salt, the crystal size and the crystal size distribution can be preferably controlled. At the same time, the characteristics as the light-sensitive material can be more improved.

In the reaction vessel, the solvent may be previously contained, and as the solvent previously placed therein, water is preferably used. However, the above-mentioned mixed solvent with the tertiary alcohol is also preferably used.

As the method of addition to the solution in the reaction vessel, in the case of the above-mentioned simultaneous addition method, it is preferred that the positions of the addition nozzles are not in close proximity to each other, and the arrangement symmetrical centered on the stirring shaft is preferred. The distance between the nozzles and the stirring shaft varies depending on the bulk mixing force of stirring, the addition speed of the addition solutions and the temperature of the reaction vessel, and there is no particular limitation thereon. According to this method, the sedimentation speed of fine crystals of the above-mentioned fatty acid alkali metal salt and the formation speed of the silver salt can be preferably established. In the case of addition to the aqueous solution containing the water-soluble silver salt which is previously wholly present in the reaction vessel, there is no particular limitation on the position of the nozzle for adding the aqueous solution of the tertiary alcohol containing the fatty acid alkali metal salt.

There is no particular limitation on the height of the tip of the nozzle for adding the aqueous solution of the tertiary alcohol containing the fatty acid alkali metal salt, as long as the tip does not come into contact with the liquid surface of the reaction solution. It is also preferred that the tip of the addition nozzle is raised with a rise of a liquid surface during the reaction. The tip of the addition nozzle outside the reaction solution not only prevents the aqueous solution of the tertiary alcohol containing the fatty acid alkali metal salt from being crystallized and solidified in the addition nozzle pipe, but also is preferred in mixing with a bulk solution. The sedimentation speed of fine crystals of the fatty acid alkali metal salt and the formation speed of the silver salt can be preferably established. Further, it is also preferred that the nozzle for adding the aqueous solution containing the water-soluble silver salt does not come into contact with the liquid surface of the reaction solution.

For forming the fatty acid silver salt particles of the present invention, there are various approaches. For obtaining the particles of the present invention, it is preferred that the solubility of the fatty acid salt in the reaction field is decreased. Further, our studies have revealed that the particles of the present invention are reduced in size with an increase in reaction time. The optimum reaction time is required to be determined by trial and error.

There is no limitation on apparatuses used in the formation of the silver salts. In particular, as stirring apparatuses, every systems, for example, bulk stirring type such as Foudler type agitator, anchor blades and paddle blades, emulsion dispersing type such as dissolvers and homogenizers, or combinations thereof, can be used. Further, as the solvent previously placed in the reaction vessel, water is used, but the mixed solution with the tertiary alcohol used in the silver ion-containing solution and the solution of the alkali metal salt of the fatty acid is also used.

As addition systems of the silver ion-containing solution and the solution of the alkali metal salt of the fatty acid, every systems such as addition onto a liquid surface, addition into liquid by insertion of an addition nozzle into liquid, shower addition and addition into a reaction field provided in a reaction solution can be used.

Further, in the present invention, for further decreasing fog and preventing an increase in fog with time to improve the image keeping stability after image formation, when the aqueous solution of the tertiary alcohol containing the alkali metal salt of the fatty acid is added into the reaction vessel, the nozzle for adding the above-mentioned aqueous solution of the tertiary alcohol is preferably attached at such a height that the tip thereof does not come into contact with the solution in the reaction vessel, as described later.

Furthermore, in the path for introducing the aqueous solution of the tertiary alcohol containing the fatty acid alkali metal salt from the preparation vessel thereof to the nozzle for adding it into the reaction vessel, the above-mentioned aqueous solution of the tertiary alcohol is preferably kept at a constant temperature of 65°C to 85°C, as

described later. When the photo thermographic light-sensitive material is prepared, this provides the effects of further decreasing fog, further preventing an increase in fog with time, and further improving the image keeping stability, and can prevent crystallization and solidification caused by a decrease in temperature of the above-mentioned aqueous solution of the tertiary alcohol, which is advantageous also with respect to the production thereof.

The time of addition of the silver ion-containing solution and the solution of the alkali metal salt of the fatty acid can also be arbitrarily selected. For example, they can be added by a method of adding them at a constant addition speed, or an accelerated addition method or decelerated addition method according to any function of time.

In order to prevent the phenomena of crystallization and solidification of the alkali metal salt of the fatty acid in the pipe path through which the alkali metal salt of the fatty acid is added from the preparation vessel thereof into the reaction vessel, the pipe path is preferably kept hot at a constant temperature within the above-mentioned temperature range.

The methods for keeping a contact temperature preferably used include a method of passing the aqueous solution of the tertiary alcohol containing the fatty acid alkali metal salt through the inside of a double pipe and passing hot water through the outside thereof, a steamed jacket method through the outside of a pipe and a method of lagging the outside of a pipe with a ribbon heater, and it is preferred that the above-mentioned aqueous solution of the tertiary alcohol containing the fatty acid alkali metal salt is kept hot so that the temperature thereof does not deviate from an established temperature.

Aqueous medium-soluble dispersing aids can be added to the silver ion-containing solution and the solution of the alkali metal salt of the fatty acid or the reaction solution used in the present invention. The dispersing aids may be any, as long as they can disperse the fatty acid silver salt formed. Specific examples thereof are based on a description of dispersing aids for the fatty acid silver salt given later.

The method for producing the fatty acid silver salt of the present invention will be illustrated with reference to Figs. 1 to 4.

Fig. 1 is a schematic view showing one embodiment of an apparatus used for producing the fatty acid silver salt. As shown in Fig. 1, an apparatus 1 comprises a preparation vessel 11 for preparing an aqueous solution A1 of a water-soluble silver salt and a preparation vessel for preparing an aqueous solution A2 of a tertiary alcohol containing a fatty acid alkali metal salt, and thermometers 13 and 14 for measuring the liquid temperature are mounted in the respective vessels 11 and 12. The liquid temperature is controlled to a specified temperature by an appropriate means based on temperatures measured by the thermometers 13 and 14.

The aqueous solution A1 of the water-soluble silver salt in the vessel 11 through a pipe 15 and the aqueous solution A2 of a tertiary alcohol containing a fatty acid alkali metal salt in the vessel 12 through a pipe 16 are introduced, and both solutions are reacted with each other in the reaction vessel 17, thereby forming the fatty acid silver salt. A thermometer 18 for measuring the liquid temperature of a solution A3 introduced is mounted in the reaction vessel 17. The temperature of the solution in the reaction vessel 17 is controlled to a specified temperature by an appropriate means based on this temperature measured. The reaction vessel 17 is equipped with a stirring means 20 comprising stirring blades 18 and a stirring shaft 19 for stirring the introduced solution to accelerate the reaction.

For preventing the temperature fluctuations of the aqueous solution A1 of the water-soluble silver salt during transfer through the pipe 15, the pipe 15 has a double pipe structure in which a pipe is further provided outside, and water W is circulated through the outer pipe in the direction indicated by the arrow. On the other hand, for preventing the temperature fluctuations of the aqueous solution A2 of the tertiary alcohol containing the fatty acid alkali metal salt during transfer, the pipe 16 is a heating pipe by steam S. The steam S is introduced from the direction indicated by the arrow in the figure through a valve 21. Further, temperature sensors 22 and 23 for detecting the respective liquid temperatures around outlets are each attached to tips of the pipes 15 and 16, respectively, and the circulation degree of water W and the opening of the valve for steam S are controlled based on the temperatures measured.

The respective tips of the pipes 15 and 16 form nozzles 151 and 161, respectively. In this case, it is preferred that the nozzles 151 and 161 are attached at such a height that they do not come into contact with the solution A3 in the reaction vessel 17, that is to say, at positions higher than a liquid surface of the solution A3. For the above-mentioned various reasons, such a construction is advantageous compared with the construction in which the nozzles 151 and 161 are fitted in the solution A3 in the reaction vessel 17 as shown in Fig. 2.

Further, in the case of the above-mentioned simultaneous addition, the nozzles 151 and 161 are preferably attached to positions apart from each other, rather than attached to adjacent positions, as described above. For example, when the attached positions of the nozzles 151 and 161 are seen centered on the attached position of the stirring shaft 19 of the stirring means 20, a symmetrical arrangement as shown in Fig. 3 is better than an adjacent arrangement as shown in Fig. 4.

The apparatus used for carrying out the production method of the present invention is illustrated with reference to embodiments shown in the figures, but is not limited thereto.

The shape of the fatty acid silver salt particles which can be used in the present invention is required to be a shape (hereinafter also referred to as a scale shape) satisfying the following characteristics:

(1) The average equivalent-sphere diameter of the fatty acid silver salt particles is from 0.1 μm to 0.8 μm;
(2) The average ratio of long sides/short sides in main planes of the fatty acid silver salt particles is from 1 to 4;
(3) The average aspect ratio of the particle is form 2 to 30; and
(4) The average thickness of the particles is from 0.01 μm to 0.20 μm.

The scale shape of the present invention is generally known as behenic acid particles. For example, this is different from particles apparently having a needle-like shape which has short and long axes as described in Handbook of Imaging Materials, Fig. 2.2 on page 45, edited by A. S. Diamond, published by Marcel Dekker (1991), and particles having no selective growing direction as described in JP-A-9-127643.

The equivalent-sphere diameter of the fatty acid silver salt particles prepared in the present invention is from 0.1 μm to 0.8 μm, and preferably from 0.1 μm to 0.6 μm.

Our studies have revealed that the particle size of the organic silver salt has an optimum particle size region, and that the equivalent-sphere diameter is required to be from 0.1 μm to 0.8 μm, for compatibility of keeping quality with light transmission properties. The above-mentioned behenic acid particles described in JP-A-9-127643 are very small in particle size, so that they are unsuitable for practical use according to our studies. Further, the particles of the present invention grow anisotropically in two-dimensional directions, and therefore, are different from the above-mentioned particles described in JP-A-9-127643, in which the particles are clearly described to have no selective growing direction.

The average ratio of long sides/short sides in main planes of the fatty acid silver salt particles of the present invention is from 1 to 4, preferably from 1 to 3, and particularly preferably from 1 to 2. The term "long side" means the longest length in a main plane, and the term "short side" means the longest length crossing the long side at right angles. Further, the average of the aspect ratios (particle sizes in main planes (equivalent-sphere diameters) /particle thicknesses) of the particles is from 2 to 30, and preferably from 2 to 15. Furthermore, average particle thickness is from 0.01 μm to 0.20 μm, and preferably from 0.01 μm to 0.15 μm.

In the fatty acid silver salt particles of the present invention, the particle size distribution is preferably monodispersed. Taking 100 times a value of the standard deviation of particle diameters divided by an average particle diameter as the coefficient of variation, the coefficient of variation of the fatty acid silver salt particles is preferably 40% or less, more preferably 20% or less, still more preferably 18% or less, and particularly preferably 15% or less. The lower limit of this coefficient of variation is about 2%, and preferably about 5%. This can be determined, for example, from particle sizes (volume weighted average diameters) determined by irradiating laser light to the organic silver salt dispersed in a solution and determining the autocorrelation function to changes in fluctuation of its scattered light with time.

From the viewpoint of the production cost, it is preferred that the maximum concentration of the fatty acid silver salt during particle formation in the formation of the fatty acid silver salt particles can be increased. It has become clear that a reduction in structural viscosity by formation of the particles in the scale shape and nucleation and grain growth at low temperature are effective for increasing the concentration of the fatty acid silver salt. In the present invention, the incorporation of both techniques makes it possible to form the particles at a concentration of 0.12 mol/liter to 0.5 mol/liter, preferably 0.14 mol/liter to 0.5 mol/liter.

In the redispersing method of the fatty acid silver salts of the present invention, it is preferred that desalting and dehydration are conducted after formation of the silver salts, in terms of a reduction in fog and prevention of film forming inhibition after coating. There is no particular limitation on these methods, and methods well known in the art can be used. Known filtration methods such as centrifugal filtration, suction filtration, ultrafiltration and flocculation washing by aggregation can be preferably used. Further, supernatant removal by centrifugal sedimentation is also preferably used. Desalting and dehydration may be conducted once or repeated twice or more. Addition and removal of water may be carried out continuously or separately. Desalting and dehydration are conducted to a degree that the conductivity of water finally dehydrated shows preferably 300 μS/cm or less, more preferably 100 μS/cm or less, and most preferably 60 μS or less. Although there is no particular limitation on the lower limit of the conductivity in this case, it is usually about 5 μS/cm.

For obtaining small-sized fine particles with no coagulation, the fatty acid silver salts which can be used in the present invention are preferably dispersed by use of dispersing agents to form fine solid particle dispersions. In preparing the fine solid particle dispersions, it is preferred that particles formed are not destroyed and only coagulation disappears. This state can be judged by comparing a TEM photograph of the particles before washing with a TEM photograph thereof after dispersion. In the particles of the present invention, when the average particle size before washing is compared with that after dispersion, it is preferred that the projected area does not change 30% or more, more preferably 20% or more, particularly preferably 10% or more. As the redispersing method, a method is preferably used in which the fatty acid silver salt desalted and dehydrated is crudely dispersed in an aqueous solvent again to prepare a crude dispersion, and then, the crude dispersion is converted to a high-pressure high-speed stream, followed by a reduction in pressure.

As this aqueous solvent, water is usually used, but a small amount (20% by weight or less) of miscible organic solvent may be added thereto.

JP-B-7-119953 (the term "JP-B" as used herein means an "examined Japanese patent publication"), JP-A-8-137044 and JP-A-8-238848 disclose methods for finely dispersing fatty acid silver salts by pressure treatment. These relate to dispersions in which organic solvents are used as solvents, and are different from the solution of the above-mentioned problems in character.

In the present invention, the coexistence of a light-sensitive silver salt in redispersing the particles results in an increase in fog and significant deterioration of sensitivity. Accordingly, it is more preferred that a light-sensitive silver salt is not substantially contained in redispersing the particles. The amount of the light-sensitive silver salt contained in an aqueous dispersion in which the particles are redispersed in the present invention is preferably 0.1 mol% or less per mol of fatty acid silver salt in the dispersion, and the light-sensitive silver salt is not positively added.

In the present invention, dispersing devices used for conducting the redispersing methods as described above and techniques thereof are described in detail in, for example, Toshio Kajiuchi and Hiromoto Usui, Dispersion System Rheology And Dispersing Technology, pages 357 to 403, Shinzansha Shuppan Co. (1991), Progress in Chemical Engineering, the 24th series, edited by Corporate Juridical Person, Kagaku Kogaku-Kai, Tokai Branch, pages 184 to 185, Maki Shoten (1990), JP-A-59-49832, U.S. Patent 4,533,254, JP-A-8-137044, JP-A-8-238848, JP-A-2-261525 and JP-A-1-94933. However, the redispersing method according to the present invention is a method of pressurizing the aqueous dispersion containing at least the fatty acid silver salt by use of a high pressure pump to supply it into a pipe, passing it through a narrow slit formed in the pipe, and thereafter, allowing a rapid reduction in pressure to occur in the dispersion, thereby performing fine dispersion.

With respect to high pressure homogenizers to which the present invention is related, dispersion to fine particles is generally considered to be carried out by dispersing force such as (a) "shearing force" developed when a dispersoid passes through a narrow slit at high pressure and speed and (b) "cavitation force" developed when the dispersoid is released from high pressure to atmospheric pressure. Dispersing devices of this kind formerly include a Gaulin homogenizer. In this device, a solution to be dispersed which is transferred at high pressure is converted to a high-speed stream at a narrow slit on a column face, and collides with a surrounding wall surface by its power. Emulsification and dispersion are conducted by this impact force. The pressure used generally ranges from 100 kg/cm$^2$ to 600 kg/cm$^2$, and the flow rate ranges from several meters/second to 30 m/second. For increasing the dispersing efficiency, a device has also been devised in which a high speed flow portion is formed in a saw teeth shape to increase the number of collisions. In contrast, devices have recently been developed in which dispersion at higher pressure becomes possible. Typical examples thereof include Microfluidizer (manufactured by Microfluidex International Corporation) and Nanomizer (manufactured by Tokushukika Kogyo Co.).

The fatty acid silver salts of the present invention can be dispersed to a desired particle size by controlling the flow rate, the pressure difference in lowering the pressure and the number of times that the treatment is conducted. However, in respect to the photographic characteristics and the particle size, the flow rate preferably ranges from 200 m/seconds to 600 m/seconds, and the pressure difference in lowering the pressure preferably ranges from 900 kg/cm$^2$ to 3000 kg/cm$^2$. More preferably, the flow rate ranges from 300 m/seconds to 600 m/seconds, and the pressure difference in lowering the pressure ranges from 1500 kg/cm$^2$ to 3000 kg/cm$^2$. The number of times that the dispersing treatment is conducted can be selected as so desired. Usually, the range of once to 10 times is selected. However, from the viewpoint of productivity, the range of once to 3 times is selected. From the viewpoints of the dispersibility and the photographic characteristics, it is unfavorable to elevate the temperature of such aqueous dispersions to high temperature under high pressure. At a temperature as high as exceeding 90°C, the particle size is liable to become large, and fog tends to increase. Accordingly, in the present invention, a step before the above-mentioned conversion to the high-pressure high-speed stream, a step after the pressure is lowered, or both steps contain a cooling device, and the temperature of such aqueous dispersions are maintained preferably at 5°C to 90°C, more preferably at 5°C to 80°C, particularly at 5°C to 60°C, by the cooling device. In particular, on dispersion at a high pressure ranging from 1500 kg/cm$^2$ to 3000 kg/cm$^2$, it is effective to provide the above-mentioned cooling device. As the cooling device, a double pipe or a triple pipe equipped with a static mixer, a multipipe heat exchanger or a coiled heat exchanger can be appropriately selected according to a required heat exchange amount thereof. Further, for enhancing the efficiency of heat exchange, the suitable size, wall thickness and material of the pipe may be selected considering the pressure used. As a refrigerant used in the cooling device, well water of 20°C, cold water of 5°C to 10°C treated with a refrigerator or a refrigerant such as ethylene glycol/water of -30°C as so desired can be used.

When the fatty acid silver salts are dispersed by use of the dispersing agents to form fine solid particles, the dispersing agents can be used which are appropriately selected from synthetic anionic polymers such as polyacrylic acid, acrylic copolymers, maleic copolymers, maleic monoester copolymers and acryloylmethylpropane-sulfonic acid copolymers, semi-synthetic anionic polymers such as carboxymethyl starch and carboxymethyl cellulose, anionic polymers such as alginic acid and pectic acid, anionic surfactants described in JP-A-52-92716 and WO88/04794, compounds described in Japanese Patent Application No. 7-350753, well-known anionic, nonionic and cationic surfactants, well-known polymers such as polyvinyl alcohol, polyvinyl pyrrolidone, hydroxypropyl cellulose and hydroxypropylmethyl cellulose, and natural polymer compounds such as gelatin.

The dispersing acid is generally mixed with a powder or a wet cake of the fatty acid silver salt before dispersion, and the resulting mixture is supplied to a dispersing device. However, the dispersing aid may be previously mixed with the fatty acid silver salt, and the resulting mixture may be heat treated or treated with a solvent to form a powder or a wet cake of the fatty acid silver salt. The pH may be controlled with an appropriate pH adjusting agent before, after or during dispersion.

In addition to mechanical dispersion, the fatty acid silver salt may be roughly dispersed in a solvent by pH control, and then, the pH may be changed in the presence of the dispersing aid to form finely divided particles. In this case, an fatty acid solvent may be used for dispersion, and usually removed after formation of the finely divided particles.

The dispersions thus prepared can be stored with stirring or in a high viscous state using hydrophilic colloids (for example, in a jelly-like state using gelatin), for inhibiting precipitation of the fine particles in storage. Further, in order to prevent the propagation of bacteria in storage, preservatives can also be added.

The particle size (volume weighted average diameter) of the dispersion of the fine solid fatty acid silver salt particles of the present invention can be determined, for example, from the particle size (volume weighted average diameter) determined by irradiating laser light to the organic silver salt dispersed in a solution and determining the autocorrelation function to changes in fluctuation of its scattered light with time.

The particle size distribution of the dispersion of the fine solid fatty acid silver salt particles is preferably monodispersed. Specifically, the percentage of a value of the standard deviation of the volume weighted average diameters divided by the volume weighted average diameter (the coefficient of variation) is preferably 40% or less, more preferably 20% or less, still more preferably 18% or less, and yet still more preferably 15% or less.

The dispersion of the fine solid fatty acid silver salt particles of the present invention comprises at least the fatty acid silver salt and water. Although there is no particular limitation on the ratio of the fatty acid silver salt to water, the ratio of the fatty acid silver salt to the whole dispersion is preferably from 5% to 50% by weight, and particularly preferably from 10% to 35% by weight.

In dispersing the particles, the above-mentioned dispersing aids are preferably used. It is preferred that they are used in minimum amounts within the range suitable for minimizing the particle size. They are preferably used within the range of 1% to 30% by weight, particularly within the range of 3% to 15% by weight, based on fatty acid silver salt.

In the present invention, the metal ion selected from the group consisting of Ca, Mg, Ce, Al, Zn and Ba is added preferably in a form of a water-soluble salt which is not a halide, whereby the image keeping quality after image formation can be further improved. The addition thereof in a halide form causes an increase in fog and deterioration of the image keeping quality.

In the present invention, the metal ion selected from the group consisting of Ca, Mg, Ce, Al, Zn and Ba may be added at any time such as previous addition to solution (1) used in the method for preparing the fatty acid silver salt in the present invention or the reaction solution, during formation of the fatty acid silver salt, immediately after formation, before dispersion, after dispersion, or before and after preparation of the coating solution, as long as it is added before coating. Preferably, it is added immediately after formation of the fatty acid silver salt, before dispersion.

The amount of these metal ions added is preferably from $10^{-3}$ mol to $10^{-1}$ mol, and particularly preferably from $5 \times 10^{-3}$ mol to $5 \times 10^{-2}$ mol, per mol of fatty acid silver salt.

As to the use of the metal salts in the light-sensitive materials, GB-1,498,406 describes that calcium behenate or magnesium behenate is used in combination at the time of coating, JP-A-47-319 describes that an acetate compound of zinc, cadmium or copper is added at the time of coating, and JP-A-51-51323 describes the use of palladium. However, these prior art techniques are strictly based on the condition that coating solutions in which organic solvents are used as solvents are applied, and the methods themselves are impossible or ineffective in the aqueous solvent systems targeted in the present invention. JP-A-52-24520 describes that cerium compounds are added in preparing silver salts, thereby improving the raw stock storability and the image discoloration resistance. However, this prior art also describes the effect obtained in the fatty acid silver salts prepared by the conventional methods, based on the condition that coating solutions using organic solvents are applied. Application to the fatty acid silver salts prepared by the conventional methods is little effective against the image discoloration resistance of light-sensitive materials to light (indoor light or sunlight). Further, addition of these metal salts in the form of halides further significantly impairs the above-mentioned characteristics, resulting in only production of products low in commercial value. Addition of the above-mentioned metal ions solves this problem.

The fatty acid silver salts prepared by the redispersing method of the present invention are dispersed in the aqueous solvents, and thereafter, mixed with aqueous solutions of light-sensitive silver salts. The solutions thus obtained are supplied as coating solutions for light-sensitive image forming media.

There is no particular limitation on the composition of the light-sensitive silver halides used in the present invention, and silver chloride, silver chlorobromide, silver bromide, silver iodobromide and silver iodochlorobromide can be used. The distribution of the halogen composition in the grain may be uniform, vary stepwise, or vary continuously. Further, silver halide grains having the core/shell structure can be preferably used. Double to fivefold structure type core/shell grains can be preferably used, and double to fourfold structure type core/shell grains can be more preferably

used. Furthermore, silver bromide can be preferably localized on the surfaces of silver chloride or silver chlorobromide grains.

Methods for forming the light-sensitive silver halides are well-known in the art. For example, methods described in Research Disclosure, vol. 17029 (June, 1978) and U.S. Patent 3,700,458 can be used. Specifically, a method is used in which light-sensitive silver halide grains are prepared by adding a silver supplying compound and a halogen supplying compound to a gelatin solution or another polymer solution, and then, the resulting silver halide grains are mixed with an organic silver salt. For inhibiting white turbidity after image formation,.it is preferred that the grain size of the light-sensitive silver halide is small. Specifically, the grain size is preferably 0.20 μm or less, more preferably from 0.01 μm to 0.15 μm, and still more preferably from 0.02 μm to 0.12 μm. The term "grain size" as used herein means the length of an edge of a silver halide grain, when the silver halide grain is a normal crystal such as a cube or octahedron. When the silver halide grain is a tabular grain, the grains size means the diameter of a corresponding circle having the same area as a projected area of a main surface. Besides, when the grain is not a normal crystal, such as a spherical or rod-like grain, the grain size means the diameter of a corresponding sphere having the same volume as that of the silver halide grain.

The form of the silver halide grains may be cubic, octahedral, tabular, spherical, rod-like or pebble-like. In the present invention, however, cubic or tabular grains are particularly preferred. When the tabular silver halide grains are used, the mean aspect ratio thereof is preferably from 100:1 to 2:1, and more preferably from 50:1 to 3:1. Further, silver halide grains having rounded corners can also be preferably used. There is no particular limitation on the surface index (mirror index) of outer surfaces of the light-sensitive silver halide grains. However, it is preferred that the ratio of the [100] face is high, said [100] face having high spectral sensitization efficiency when a spectral sensitizing dye is adsorbed thereby. The ratio is preferably 50% or more, more preferably 65% or more, and most preferably 80% or more. The ratio of the [100] face can be determined by a method described in T. Tani, Imaging Sci., 29, 165 (1985), utilizing adsorption dependency of the [111] face and the [100] face in adsorption of a sensitizing dye.

The light-sensitive silver halide grains of the present invention contain a metal or metal complex of group VII or VIII in the periodic table. The metals or main metals of the metal complexes of group VII or VIII in the periodic table are rhodium, rhenium, ruthenium, osmium and iridium. These metal complexes may be used either alone or as a combination of two or more of the same kind or different kinds of complexes. The content thereof is preferably from $1\times10^{-9}$ mol to $1\times10^{-3}$ mol, and more preferably from $1\times10^{-8}$ mol to $1\times10^{-3}$ mol, per mol of silver. As to the specific structures thereof, metal complexes having structures described in JP-A-7-225449 can be used.

As the rhodium compounds used in the present invention, water-soluble rhodium compounds can be used. Examples thereof include rhodium (III) halide compounds or rhodium complex salts having halogens, amines, oxalato or the like as ligands, for example, hexachlororhodium (III) complex salts, pentachloroaquorhodium (III) complex salts, tetrachloro-diaquorhodium (III) complex salts, hexabromorhodium (III) complex salts, hexaamminerhodium (III) complex salts and trioxalatorhodium (III) complex salts. These rhodium compounds are used by dissolving them in water or appropriate solvents. In order to stabilize the solution of the rhodium compound, a method of adding an aqueous solution of a hydrogen halide (for example, hydrochloric acid, hydrobromic acid or hydrofluoric acid) or an alkali halide (for example, KCl, NaCl, KBr or NaBr), which is generally frequently used, can be used. Instead of use of the water-soluble rhodium, it is also possible to add and dissolve other silver halide grains previously doped with rhodium in preparing the silver halide.

The amount of these rhodium compounds added is preferably from $1\times10^{-8}$ mol to $5\times10^{-6}$ mol, and particularly preferably from $5\times10^{-8}$ mol to $1\times10^{-6}$ mol, per mol of silver halide.

These compounds can be appropriately added in preparing the silver halide emulsion grains and in each stage prior to coating of the emulsions. In particular, the compounds are preferably added in forming the emulsions to incorporate them into the silver halide grains.

Rhenium, ruthenium and osmium used in the present invention are added in the form of water-soluble complex salts described in JP-A-63-2042, JP-A-1-285941, JP-A-2-20852 and JP-A-2-20855. Particularly preferred examples thereof include six-coordinate complexes represented by the following formula:

$$[ML_6]^{n-}$$

wherein M represents Ru, Re or Os, L represents a ligand, and n represents 0, 1, 2, 3 or 4.

In this case, counter ions have no importance, and ammonium or alkali metal ions are used as the counter ions.

Preferred examples of ligands include halide ligands, cyanide ligands, cyanate ligands, nitrosyl ligands and thionitrocyl ligands. Specific examples of the complexes used in the present invention are shown below, but the scope of the invention is not limited thereto.

$[ReCl_6]^{3-}$, $[ReBr_6]^{3-}$, $[ReCl_5(NO)]^{2-}$, $[Re(NS)Br_5]^{2-}$, $[Re(NO)(CN)_5]^{2-}$, $[Re(O)_2(CN)_4]^{3-}$, $[RuCl_6]^{3-}$, $[RuCl_4(H_2O)_2]^{1-}$, $[RuCl_5(H_2O)]^{2-}$, $[RuCl_5(NO)]^{2-}$, $[RuBr_5(NS)]^{2-}$, $[Ru(CO)_3Cl_3]^{2-}$, $[Ru(CO)Cl_5]^{2-}$, $[Ru(CO)Br_5]^{2-}$, $[OsCl_6]^{3-}$

, $[OsCl_5(NO)]^{2-}$, $[Os(NO)(CN)_5]^{2-}$, $[Os(NS)Br_5]^{2-}$, $[Os(O)_2(CN)_4]^{4-}$

The amount of these compounds added is preferably from $1\times10^{-9}$ mol to $1\times10^{-5}$ mol, and particularly preferably from $1\times10^{-8}$ mol to $1\times10^{-6}$ mol, per mol of silver halide.

These compounds can be appropriately added in preparing the silver halide emulsion grains and in each stage prior to coating of the emulsions. In particular, the compounds are preferably added in forming the emulsions to incorporate them into the silver halide grains.

Methods for adding these compounds in forming the silver halide grains to incorporate them into the silver halide grains include a method of adding a metal complex powder or an aqueous solution in which the compound is dissolved together with NaCl and KCl to a water-soluble salt or a water-soluble halide solution during grain formation, a method of adding the compound as the third solution, when a silver salt and a halide solution are simultaneously mixed, to prepare the silver halide grains by simultaneous mixing of three solutions, and a method of putting a necessary amount of an aqueous solution of the metal complex into a reaction vessel during grain formation. In particular, the method of adding the powder or the aqueous solution in which the compound is dissolved together with NaCl and KCl to the water-soluble halide solution is preferred.

For adding the compound to the surfaces of the grains, a necessary amount of an aqueous solution of the metal complex can also be put into a reaction vessel immediately after grain formation, during or at the end of physical ripening, or in chemical ripening.

As the iridium compounds used in the present invention, various iridium compounds can be used. Examples thereof include hexachloroiridium, hexaammineiridium, trioxalato-iridium, hexacyanoiridium and pentachloronitrosyliridium. These iridium compounds are used by dissolving them in water or appropriate solvents. In order to stabilize the solution of the iridium compound, a method of adding an aqueous solution of a hydrogen halide (for example, hydrochloric acid, hydrobromic acid or hydrofluoric acid) or an alkali halide (for example, KCl, NaCl, KBr or NaBr), which is generally frequently used, can be used. Instead of use of the water-soluble iridium, it is also possible to add and dissolve other silver halide grains previously doped with iridium in preparing the silver halide.

The silver halide grains used in the present invention may further contain metal atoms such as cobalt, iron, nickel, chromium, palladium, platinum, gold, thallium, copper and lead. As to compounds of cobalt, iron, chromium and further ruthenium, hexacyano metal complexes can be preferably used. Specific examples thereof include but are not limited to a ferricyanate ion, a ferrocyanate ion, a hexacyanocobaltate ion, a hexacyanochromate ion and a hexacyanoruthenate ion. There is no particular limitation on a metal complex-containing phase in the silver halide grain. The metal complex may be uniformly contained in the grain, or may be contained at a high concentration in a core portion or a shell portion.

The above-mentioned metals are preferably contained in an amount of $1\times10^{-9}$ mol to $1\times10^{-4}$ mol per mol of silver halide. For adding the above-mentioned metals, they can be added as metal salts such as single salts, double salts or complex salts, in preparing the grains.

The light-sensitive silver halide grains can be desalted by washing according to methods well-known in the art, such as the noodle method and the flocculation method. In the present invention, however, the silver halide grains may be desalted or not.

Gold sensitizers used at the time when the silver halide emulsions are subjected to gold sensitization in the present invention may have an oxidation number of plus mono- or plus tri-valency, and gold compounds usually employed as the gold sensitizers can be used. Typical examples thereof include chloroauric acid, potassium chloroaurate, auric trichloride, potassium auric thiocyanate, potassium iodoaurate, tetracyanoauric acid, ammonium aurothiocyanate and pyridyltrichlorogold.

Although the amount of the gold sensitizers added varies depending on various conditions, it is from $1\times10^{-7}$ mol to $1\times10^{-3}$ mol, and preferably from $1\times10^{-6}$ mol to $5\times10^{-4}$ mol, per mol of silver halide, as a guide.

When the silver halide emulsions used in the present invention are sensitized, gold sensitization is preferably used in combination with different chemical sensitization. As the different chemical sensitization methods, well-known methods such as sulfur sensitization, selenium sensitization, tellurium sensitization and noble metal sensitization can be used. When they are used in combination with gold sanitization, for example, a combination of sulfur sensitization and gold sensitization, a combination of selenium sensitization and gold sensitization, a combination of sulfur sensitization, selenium sensitization and gold sensitization, a combination of sulfur sensitization, tellurium sensitization and gold sensitization, and a combination of sulfur sensitization, selenium sensitization, tellurium sensitization and gold sensitization are preferred.

The sulfur sensitization preferably used in the present invention is usually conducted by adding a sulfur sensitizer and stirring an emulsion at a high temperature of 40°C or more for a definite period of time. As the sulfur sensitizers, well-known compounds can be used. Examples thereof include various sulfur compounds such as thiosulfates, thiourea compounds, thiazole compounds and rhodanine compounds, as well as sulfur compounds contained in gelatin. Preferred sulfur compounds are thiosulfates and thiourea compounds. Although the amount of the sulfur sensitizers added varies depending on various conditions such as the pH and the temperature in chemical ripening, and the size

of silver halide grains, it is from $1\times10^{-7}$ mol to $1\times10^{-2}$ mol, and more preferably from $1\times10^{-5}$ mol to $1\times10^{-3}$ mol, per mol of silver halide.

As selenium sensitizers used in the present invention, well-known selenium compounds can be used. That is to say, the selenium sensitization is usually conducted by adding a labile type and/or non-labile type selenium compound and stirring an emulsion at a high temperature of 40°C or more for a definite period of time. As the labile type selenium compounds, compounds described in JP-B-44-15748, JP-B-43-13489, JP-A-4-25832, JP-A-4-109240 and JP-A-3-121798 can be used. In particular, compounds represented by formulas (VIII) and (IX) in JP-A-4-324855 are preferably used.

Tellurium sensitizers used in the present invention are compounds producing silver telluride presumed to form a sensitizing nucleus on a surface or in the inside of a silver halide grain. The forming rate of silver telluride in the silver halide emulsion can be tested by a method described in JP-A-5-313284. Examples of the tellurium sensitizers include diacyl tellurides, bis(oxycarbonyl) tellurides, bis (carbamoyl) tellurides, bis (oxycarbonyl) ditellurides, bis(carbamoyl) ditellurides, P=Te bond-containing compounds, tellurocarboxylic acid esters, Te-organyltelluro carboxylic acid esters, di(poly)tellurides, tellurides, tellurols, telluroacetals, tellurosulfonates, P-Te bond-containing compounds, Te-containing heterocyclic compounds, tellurocarbonyl compounds, inorganic tellurium compounds and colloidal tellurium. Specifically, compounds can be used which are described in U.S. Patents 1,623,499, 3,320,069 and 3,772,031, GB-235,211, GB-1,121,496, GB-1,295,462, GB-1,396,696, Canadian Patent 800,958, JP-A-4-204640, JP-A-3-53693, JP-A-3-131598, JP-A-4-129787, J. Chem. Soc. Chem. Commun., 635 (1980), ibid., 1102 (1979), ibid., 645 (1979), J. Chem. Soc. Perkin. Trans., 1, 2191 (1980), The Chemistry of Organic Selenium and Tellurium Compounds, vol.1, edited by S. Patai (1986) and ibid. vol. 2 (1987). In particular, compounds represented by general formulas (II), (III) and (IV) in JP-A-5-313284 are preferably used.

The amount of the selenium and tellurium sensitizers used in the present invention is generally from $1\times10^{-8}$ mol to $1\times10^{-2}$ mol, and preferably from about $1\times10^{-7}$ mol to about $1\times10^{-3}$ mol, per mol of silver halide, although it varies depending on silver halide grains used and chemical ripening conditions. There is no particular limitation on the conditions of chemical sensitization in the present invention. However, the pH is from 5 to 8, the pAg is from 6 to 11 and preferably from 7 to 10, and the temperature is from 40°C to 95°C and preferably from 45°C to 85°C.

In the course of formation of the silver halide grains and physical ripening, cadmium salts, sulfites, lead salts and thallium salts may be allowed to coexist with the silver halide emulsions used in the present invention.

In the present invention, reduction sensitization can be used. Specific examples of compounds which can be used in reduction sensitization include stannous chloride, aminoiminomethanesulfonic acid, hydrazine derivatives, borane compounds, silane compounds and polyamines, as well as ascorbic acid and thiourea dioxide. Further, reduction sensitization can be performed by ripening the emulsions while maintaining the pH of the emulsions at 7 or more, or the pAg thereof at 8.3 or less. Furthermore, reduction sensitization can be conducted by introducing single addition portions of silver ions during grain formation.

Thiosulfonic acid compounds may be added to the silver halide emulsions used in the present invention by a method shown in EP-293,917.

The silver halide emulsions in the light-sensitive materials used in the present invention may be used either alone or as a combination of two or more of them (for example, emulsions different in mean grain size, emulsions different in halogen composition, emulsions different in crystal habit, and emulsions different in the conditions of chemical sensitization).

The amount of the light-sensitive silver halides used in the present invention is preferably from 0.01 mol to 0.5 mol, more preferably from 0.02 mol to 0.3 mol, and particularly preferably from 0.03 mol to 0.25 mol, per mol of organic silver salt.

The silver halides used in the present invention are preferably added to the coating solutions for image forming layers from 180 minutes before coating to immediately before coating, preferably from 60 minutes before coating to 10 seconds before coating. However, there is no particular limitation on the mixing process and the mixing conditions, as long as the effects of the present invention are sufficiently manifested. Specific examples of the mixing processes include a mixing process using a tank designed so that the average residence time calculated from the flow rate of the solution added and the amount of the solution supplied to a coater becomes a desired time, and a process using static mixers described in N. Harnby, M. F. Edwards and A. W. Nienow, translated by Koji Takahashi, Liquid Mixing Techniques, chapter 8, published by Nikkan Kogyo Shinbunsha (1989).

It is preferred that the heat image forming materials of the present invention contain reducing agents for the organic silver salts. The reducing agents for the organic silver salts may be any substances for reducing a silver ion to metallic silver, and preferably organic substances. Although conventional photographic developing agents such as phenidone, hydroquinone and cathecol are useful, hindered phenol reducing agents are preferred. The reducing agent is contained preferably in an amount of 5 mol% to 50 mol%, and more preferably in an amount of 10 mol% to 40 mol%, per mol of silver of a face having an image formation layer. The reducing agent may be added to any layers of the face having the image formation layer. When the reducing agent is added to a layer other than the image formation layer, it

is preferably used in such a larger amount as 10 mol% to 50 mol% per mol of silver. Further, the reducing agent may be a so-called precursor which is derived so as to effectively function only in development.

A wide variety of reducing agents used in the photo thermographic light-sensitive materials employing the organic silver salts are disclosed in JP-A-46-6074, JP-A-47-1238, JP-A-47-33621, JP-A-49-46427, JP-A-49-115540, JP-A-50-14334, JP-A-50-36110, JP-A-50-147711, JP-A-51-32632, JP-A-51-1023721, JP-A-51-32324, JP-A-51-51933, JP-A-52-84727, JP-A-55-108654, JP-A-56-146133, JP-A-57-82828, JP-A-57-82829, JP-A-6-3793, U.S. Patents 3,667,958, 3,679,426, 3,751,252, 3,751,255, 3,761,270, 3,782,949, 3,839,048, 3,928,686 and 5,464,738, German Patent 2,321,328 and European Patent 692,732.

Examples thereof include amidooximes such as phenylamido-oxime, 2-thienylamidooxime and p-phenoxyphenylamidooxime; azines such as 4-hydroxy-3,5-dimethoxybenzaldehydeazine; combinations of aliphatic carboxylic acid arylhydrazides and ascorbic acid such as a combination of 2,2'-bis(hydroxy-methyl)propionyl-β-phenylhydrazine and ascorbic acid; combinations of polyhydroxybenzene and hydroxylamine compounds, reductones and/or hydrazines (for example, a combination of hydroquinone and bis(ethoxyethyl)hydroxyl-amine, piperidinohexosereductone or formyl-4-methylphenylhydrazine); hydroxamic acids such as phenylhydroxamic acid, p-hydroxyphenylhydroxamic acid and β-anilinehydroxamic acid; combinations of azines and sulfonamidophenols (for example, a combination of phenothiazine and 2,6-dichloro-4-benzene-sulfonamidophenol); α-cyanophenylacetic acid derivatives such as ethyl-α-cyano-2-methylphenyl acetate and ethyl-α-cyanophenyl acetate; bis-β-naphthols such as 2,2'-dihydroxy-1,1'-bisnaphthyl, 6,6'-dibromo-2,2'-dihydroxy-1,1'-binaphthyl and bis(2-hydroxy-1-naphthyl)methane; combinations of bis-β-naphthols and 1,3-dihydroxybenzene derivatives (for example, 2,4-dihydroxybenzophenone or 2',4'-dihydroxyacetophenone); 5-pyrazolone derivatives such as 3-methyl-1-phenyl-5-pyrazolone; reductones such as dimethylaminohexosereductone, anhydrodihydroaminohexosereductone and anhydrodihydropiperidonehexosereductone; sulfonamidophenol reducing agents such as 2,6-dichloro-4-benzenesulfonamidophenol and p-benzenesulfonamidophenol; 2-phenylindane-1,3-dione; chroman derivatives such as 2,2-dimethyl-7-t-butyl-6-hydroxychroman; 1,4-dihydropyridine derivatives such as 2,6-dimethoxy-3,5-dicarboethoxy-1,4-dihydropyridine; bisphenols (for example, bis(2-hydroxy-3-t-butyl-5-methylphenyl)methane, 2,2-bis(4-hydroxy-3-methylphenyl)propane, 4,4-ethylidene-bis(2-t-butyl-6-methylphenol), 1,1-bis(2-hydroxy-3,5-dimethylphenyl)-3,5,5-trimethylhexane and 2,2-bis(3,5-dimethyl-4-hydroxyphenyl)propane); ascorbic acid derivatives (for example, 1-ascorbyl palmitate and ascorbyl stearate); aldehydes and ketones of benzyl and biacetyl; 3-pyrazolidone and some kind of indane-1,3-dione; and chromanols (such as tocopherol). Bisphenols and chromanols are particularly preferred.

The reducing agents used in the present invention may be added in any forms such as solutions, powders or fine solid particle dispersions. The fine solid particles are dispersed by well-known finely dividing means (for example, ball mills, vibrating ball mills, sand mills, colloid mills, jet mills and roller mills). Further, dispersing aids may be used in dispersing the fine solid particles.

When an additive known as a "toning agent" for improving an image is contained, the optical density is sometimes increased. Further, the toning agent also becomes advantageous for forming a black silver image in some cases. The toning agent is contained in a face having the image formation layer preferably in an amount of 0.1 mol% to 50 mol%, and more preferably in an amount of 0.5 mol% to 20 mol%, per mol of silver. The toning agent may be a precursor which is derived so as to effectively function only in development.

A wide variety of toning agents used in the photothermographic light-sensitive materials employing the organic silver salts are disclosed in JP-A-46-6077, JP-A-47-10282, JP-A-49-5019, JP-A-49-5020, JP-A-49-91215, JP-A-50-2524, JP-A-50-32927, JP-A-50-67132, JP-A-50-67641, JP-A-50-114217, JP-A-51-3223, JP-A-51-27923, JP-A-52-14788, JP-A-52-99813, JP-A-53-1020, JP-A-53-76020, JP-A-54-156524, JP-A-54-156525, JP-A-61-183642, JP-A-4-56848, JP-B-49-10727, JP-B-54-20333, U.S. Patents 3,080,254, 3,446,648, 3,782,941, 4,123,282 and 4,510,236, GB-1,380,795 and Belgian Patent 841,910. Examples of the toning agents include phthalimide and N-hydroxyphthalimide; cyclic imides such as succinimide, pyrazoline-5-one, quinazolinone, 3-phenyl-2-pyrazoline-5-one, 1-phenylurazole, quinazoline and 2,4-thiazolidinedione; naphthalimides (for example, N-hydroxy-1,8-naphthalimide); cobalt complexes (for example, cobalthexamine trifluoroacetate); mercaptans (3-mercapto-1,2,4-triazole, 2,4-dimercaptopyrimidine, 3-mercapto-4,5-diphenyl-1,2,4-triazole and 2,5-dimercapto-1,3,4-thiadiazole; N-(aminomethyl)aryldicarboxyimides (for example, (N,N-dimethylaminomethyl)phthalimide and N,N-(dimethylamino-methyl)naphthalene-2,3-dicarboxyimide); blocked pyrazole, isothiuronium derivatives, and some kinds of light fading agents (for example, N,N'-hexamethylenebis(1-carbamoyl-3,5-dimethylpyrazole), 1,8-(3,6-diazaoctane)bis(isothiuronium trifluoroacetate) and 2-tribromomethylsulfonyl(benzo-thiazole)); 3-ethyl-5-[(3-ethyl-2-benzothiazolinylidene)-1-methylethylidene]-2-thio-2,4-oxazolidinedione; phthalazinone, phthalazinone derivatives such as 4-(1-naphthyl)-phthalazinone, 6-chloro-phthalazinone, 6-tert-butyl-phthalizinone, 6-isopropyl-phthalazinone, 5,7-dimethoxy-phthalazinone and 2,3-dihydro-1,4-phthalazinedione, or metal salts thereof; combinations of phthalazinone and phthalic acid derivatives (for example, phthalic acid, 4-methyl-phthalic acid, 4-nitrophthalic acid and tetrachlorophthalic anhydride); phthalazine, phthalazine derivatives such as 4-(1-naphthyl)phthalazine, 6-isopropyl-phthalazine, 6-tert-butyl-phthalazine, 6-chlorophthalazine, 5,7-dimethoxy-phthalazine and 2,3-dihydrophthalazine, or metal salts thereof, combinations of phthalazine and phthalic

acid derivatives (for example, phthalic acid, 4-methylphthalic acid, 4-nitrophthalic acid and tetrachlorophthalic anhydride); quinazolinedione, benzoxazine or naphthoxazine derivatives; rhodium complexes which function not only as the toning agents, but also as sources of halide ions for silver halide formation, such as ammonium hexachlororhodate (III), rhodium bromide, rhodium nitrate and potassium hexachlororhodate (III); inorganic peroxides and persulfates such as ammonium disulfide peroxide and hydrogen peroxide; benzoxazine-2,4-dione derivatives such as 1,3-benzoxazine-2,4-dione, 8-methyl-1,3-benzoxazine-2,4-dione and 6-nitro-1,3-benzoxazine-2,4-dione; pyrimidine and asymmetric triazine derivatives (for example, 2,4-dihydroxypyrimidine and 2-hydroxy-4-aminopyrimidine); and azauracil and tetraaza-pentalene derivatives (for example, 3,6-dimercapto-1,4-diphenyl-1H,4H-2,3a,5,6a-tetraazapentalene and 1,4-di (o-chlorophenyl)-3,6-dimercapto-1H,4H-2,3a,5,6a-tetraaza pentalene).

The toning agents used in the present invention may be added in any forms such as solutions, powders or fine solid particle dispersions. The fine solid particles are dispersed by well-known finely dividing means (for example, ball mills, vibrating ball mills, sand mills, colloid mills, jet mills and roller mills). Further, dispersing aids may be used in dispersing the fine solid particles.

The photothermographic light-sensitive material is usually produced by applying the coating solution for the light-insensitive layer containing the fatty acid silver salt, the reducing agent for silver ions, the binder and the light-sensitive silver halide onto a support, and drying it. However, the present invention is effective in the production method in which the coating solution for the light-sensitive layer contains 30% by weight or more of water as a solvent, a polymer dispersed in a latex form as the main binder, and the fatty acid silver salt particles dispersed by the redispersing method of the present invention as a dispersion.

In particular, when the binder comprises a polymer latex having an equilibrium moisture content at 25°C, 60% RH of 2% by weight or less, the increasing rate of fog in the photothermographic light-sensitive material of the present invention is improved. The polymer latex prepared so as to give an ionic conductivity of 0.05 mS/cm to 2.5 ms/cm is most preferred. Methods for preparing such a polymer latex include a method of purifying a polymer with an isolation membrane after synthesis.

The term "polymer-dispersible aqueous solvent" as used herein means water or a mixture of water and 70% by weight or less of a water-miscible organic solvent. The water-miscible organic solvents include, for example, alcohols such as methyl alcohol, ethyl alcohol and isopropyl alcohol, cellosolve derivatives such as methyl cellosolve, ethyl cellosolve and butyl cellosolve, ethyl acetate and dimethylformamide.

When the polymer is not dissolved thermodynamically to exist in a so-called dispersion state, the term "aqueous solvent" is also used herein.

The term "equilibrium moisture content at 25°C, 60% RH" as used in this invention can be expressed using the weight W1 of a polymer attaining equilibrium with moisture in the atmosphere of 25°C and 60% RH and the weight W0 of the polymer in the absolute dry condition at 25°C as follows:

$$\text{Equilibrium Moisture Content at } 25°C,\ 60\%\ RH = [(W1-W0)/W0] \times 100\ (\%\ \text{by weight})$$

For the definition of the moisture content and the measuring method thereof, reference can be made to Polymer Engineering Course 14, Test Methods of Polymer Materials (edited by Kobunshi Gakkai, Chijin Shokan).

In the present invention, the equilibrium moisture content at 25°C, 60% RH of the polymer is preferably 2% by weight or less, more preferably from 0.01% to 1.5% by weight, and still more preferably from 0.02% to 1% by weight.

In the present invention, there is no particular limitation on the binder, as long as it can be dissolved or dispersed in the aqueous solvent, and has an equilibrium moisture content at 25°C, 60% RH of 2% by weight or less. Of these polymers, ones dispersible in the aqueous solvents are particularly preferred.

Examples of the dispersion states include latexes with fine particles of solid polymers dispersed and dispersions of polymer molecules in a molecular state or forming micelles, and both are preferred.

Examples of the polymers which can be preferably used in the present invention include hydrophobic polymers such as acrylic resins, polyester resins, rubber resins (for example, SBR resins), polyurethane resins, vinyl chloride resins, vinyl acetate resins, vinylidene chloride resins and polyolefin resins. The polymer may be either a straight chain polymer or a branched polymer, or may be crosslinked. Further, the polymer may be either a so-called homopolymer in which a single monomer is polymerized, or a copolymer in which two or more kinds of monomers are polymerized. The copolymer may be either a random copolymer or a block copolymer. The number average molecular weight of the polymer is preferably from 5,000 to 1,000,000, and more preferably from about 10,000 to about 200,000. Too low a molecular weight unfavorably results in insufficient mechanical strength of the emulsion layer, whereas too high a molecular weight causes poor film forming properties.

These polymers used in the present invention are dispersed in aqueous dispersion media. The term "aqueous dispersion media" as used herein means dispersion media having 30% by weight or more of water in their composition. The dispersion state may be any, such as emulsified dispersion, micelle dispersion or dispersion of polymers having hydrophilic sites in their molecules in a molecular state. Of these, latexes are particularly preferred.

Preferred examples of the polymers include the following, in which the polymers are represented by raw material monomers, the numerals in parentheses are percentages by weight, and the molecular weight is the number average molecular weight.

P-1: Latex of -MMA(70)-EA(27)-MAA(3)- (molecular weight: 37,000)
P-2: Latex of -MMA(70)-2EHA(20)-St(5)-AA(5)-(molecular weight: 40,000)
P-3: Latex of -St(50)-Bu(47)-MAA(3)- (molecular weight: 45,000)
P-4: Latex of -St(68)-Bu(29)-AA(3)- (molecular weight: 60,000)
P-5: Latex of -St(70)-Bu(27)-IA(3)- (molecular weight: 120,000)
P-6: Latex of -St(75)-Bu(24)-AA(1)- (molecular weight: 108,000)
P-7: Latex of -St(60)-Bu(35)-DVB(3)-MAA(2)-(molecular weight: 150,000)
P-8: Latex of -St(70)-Bu(25)-DVB(2)-AA(3)- (molecular weight: 280,000)
P-9: Latex of -VC(50)-MMA(20)-EA(20)-AN(5)-AA(5)-(molecular weight: 80,000)
P-10: Latex of -VDC(85)-MMA(5)-EA(5)-MAA(5)-(molecular weight: 67,000)
P-11: Latex of -Et(90)-MMA(10)- (molecular weight: 12,000)

Abbreviations used in the above-mentioned structures indicate the following monomers:

MMA; Methyl methacrylate, EA; Ethyl acrylate, MAA; Methacrylic acid, 2EHA; 2-Ethylhexyl acrylate, St; Styrene, Bu; Butadiene, AA; Acrylic acid, DVB; Divinylbenzene, VC; Vinyl chloride, AN; Acrylonitrile, VDC; Vinylidene chloride, Et: Ethylene and IA; Itaconic acid

The polymers described above are commercially available, and the following polymers can be utilized. Examples of the acrylic resins include Sevian A-4635, 46583 and 4601 (manufactured by Daicel Chemical Industries, Ltd.) and Nipol Lx 811, 814, 821, 820 and 857 (manufactured by Nippon Zeon Co., Ltd), examples of the polyester resins include FINETEX ES 650, 611, 675 and 850 (manufactured by Dainippon Ink & Chemicals, Inc.) and WD-size and WMS (manufactured by Eastman Chemical Co.), examples of the polyurethane resins include HYDRAN AP 10, 20, 30 and 40 (manufactured by Dainippon Ink & Chemicals, Inc.), examples of the rubber resins include LACSTAR 7310K, 3307B, 4700H and 7132C (manufactured by Dainippon Ink & Chemicals, Inc.) and Nipol Lx 416, 410, 438C and 2507 (manufactured by Nippon Zeon Co., Ltd.), examples of the vinyl chloride resins include G351 and G576 (manufactured by Nippon Zeon Co., Ltd.), examples of the vinylidene chloride resins include L502 and L513 (manufactured by Asahi Chemical Industry Co., Ltd.), and examples of the polyolefin resins include Chemipearl S120 and SA100 (manufactured by Mitsui Petrochemical Industries, Ltd.).

These polymers may be used as latexes either alone or as a mixture of two or more of them as required.

As the polymer latexes used in the present invention, styrene-butadiene copolymer latexes are particularly preferred. In the styrene-butadiene copolymer latex, the weight ratio of styrene monomer units to butadiene monomer units is preferably from 40:60 to 95:5. Further, the ratio of the styrene monomer units and the butadiene monomer units to the copolymer is preferably from 60% to 99% by weight. The preferred molecular weight range is the same as described above.

The styrene-butadiene copolymer latexes which can be preferably used in the present invention include P-3 to P-8 described above and commercially available LACSTAR-3307B, 7132C and Nipol Lx416.

The organic silver salt-containing layer of the light-sensitive material of the present invention may further contain a hydrophilic polymer such as gelatin, polyvinyl alcohol, methyl cellulose or hydroxypropyl cellulose. The amount of the hydrophilic polymer added is preferably 30% by weight or less, and more preferably 20% by weight or less, of the total binder of the organic silver salt-containing layer.

The organic silver salt-containing layer of the present invention is formed using the polymer latex, and in the amount of binder contained in the organic silver salt-containing layer, the weight ratio of total binder/organic silver salt is preferably from 1/10 to 10/1, and more preferably from 1/5 to 4/1.

Further, such an organic silver salt-containing layer is also usually a light-sensitive layer (emulsion layer) containing the light-sensitive silver halide, which is the light-sensitive silver salt. In such a case, the weight ratio of total binder/silver halide is preferably from 400 to 5, and more preferably from 200 to 10.

The total binder amount of the image formation layer of the present invention is preferably from 0.2 $g/m^2$ to 30 $g/m^2$ , and more preferably from 1 $g/m^2$ to 15 $g/m^2$. The image formation layers of the present invention may contain crosslinking agents for crosslinking and surfactants for improving coating properties.

In the present invention, the solvent (both the solvent and the dispersing medium are referred to as the solvent herein for brevity) for the organic silver salt-containing layer of the light-sensitive material of the present invention is an aqueous solvent containing water in an amount of 30% by weight or more. As components other than water, any water-miscible organic solvents such as methyl alcohol, ethyl alcohol, isopropyl alcohol, methyl cellosolve, ethyl cellosolve,

dimethylformamide and ethyl acetate may be used. The water content of the solvents of the coating solutions is preferably 50% by weight or more, and more preferably 70% by weight or more. Preferred examples of solvent compositions include water/methyl alcohol = 90/10, water/methyl alcohol = 70/30, water/methyl alcohol/dimethylformamide = 80/15/5, water/methyl alcohol/ethyl cellosolve = 85/10/5 and water/methyl alcohol/isopropyl alcohol =85/10/5 (wherein the numerals are percentages by weight).

Sensitizing dyes used in the present invention may be any as long as they can spectrally sensitize the silver halide grains in a desired wavelength region when adsorbed by the silver halide grains. The sensitizing dyes which can be used include cyanine dyes, merocyanine dyes, complex cyanine dyes, complex merocyanine dyes, holopolarcyanine dyes, styryl dyes, hemicyanine dyes, oxonol dyes and hemioxonol dyes. Useful sensitizing dyes used in the present invention are described, for example, in Research Disclosure, Item 17643 IV-A, page 23 (December, 1978), ibid., Item 1831 X, page 437 (August, 1979), and literatures cited therein. In particular, sensitizing dyes having spectral sensitivity suitable for spectral characteristics of light sources of various laser imagers, scanners, image setters and process cameras can be advantageously selected.

As examples of the spectral sensitizing agents to red light, compounds I-1 to I-38 described in JP-A-54-18726, compounds I-1 to I-35 described in JP-A-6-75322, compounds I-1 to I-34 described in JP-A-7-287338, dyes 1 to 20 described in JP-B-55-39818 and compounds I-1 to I-37 described in JP-A-62-284343 are advantageously selected for so-called red light sources such as He-Ne lasers, red semiconductor lasers and LEDs.

For a semiconductor laser light source having a wavelength region of 750 nm to 1400 nm, the silver halide grains can be spectrally advantageously sensitized with various well-known dyes including cyanine, merocyanine, styryl, hemicyanine, oxonol, hemioxonol and xanthene dyes. Examples of the useful cyanine dyes include cyanine dyes having basic nuclei such as thiazoline, oxazoline, pyrroline, pyridine, oxazole, thiazole, selenazole and imidazole nuclei. Preferred examples of the useful merocyanine dyes include merocyanine dyes having acidic nuclei such as thiohydantoin, rhodanine, oxazolidinedione, thiazolinedione, barbituric acid, thiazolinone, malononitrile and pyrazolone nuclei, as well as the above-mentioned basic nuclei. In the above-mentioned cyanine and merocyanine dyes, dyes having imino groups or carboxyl groups are particularly effective. For example, they may be appropriately selected from well-known dyes as described in U.S. Patents 3,761,279, 3,719,495 and 3,877,943, GB-1,466,201, GB-1,469,117 and GB-1,422,057, JP-B-3-10391, JP-B-6-52387, JP-A-5-341432, JP-A-6-194781 and JP-A-6-301141.

Structurally preferred examples of the dyes used in the present invention include cyanine dyes having thioether bond-containing substituent groups (for example, dyes described in JP-A-62-58239, 3-138638, JP-A-3-138642, JP-A-4-255840, JP-A-5-72659, JP-A-5-72661, JP-A-6-222491, JP-A-2-230506, JP-A-6-258757, JP-A-6-317868, JP-A-6-324425, JP-W-7-500926 (the term "JP-W" as used herein means an "unexamined published international patent application") and U.S. Patent 5,541,054, dyes having carboxylic acid groups (for example, dyes described in JP-A-3-163440, JP-A-6-301141 and U.S. Patent 5,441,899), and merocyanine dyes, polynuclear merocyanine dyes and polynuclear cyanine dyes (dyes described in JP-A-47-6329, JP-A-49-105524, JP-A-51-127719, JP-A-52-80829, JP-A-54-61517, JP-A-59-214846, JP-A-60-6750, JP-A-63-159841, JP-A-6-35109, JP-A-6-59381, JP-A-7-146537, JP-A-7-146537, JP-W-55-50111, GB-1,467,638 and U.S. Patent 5,281,515).

Further, J-band formable dyes are disclosed in U.S. Patent 5,510,236, example 5 of U.S. Patent 3,871,887, JP-A-2-96131 and JP-A-59-48753, and they can be preferably used in the present invention.

These sensitizing dyes may be used alone or as a combination of two or more of them. Combinations of the sensitizing dyes are often used particularly for supersensitization. The emulsions may contain dyes having no spectral sensitizing function for themselves, or substances which do not substantially absorb visible light and exhibit supersensitization, together with the sensitizing dyes.

The useful sensitizing dyes, the combinations of the dyes showing supersensitization, and the substances exhibiting supersensitization are described in Research Disclosure, 176, 17643 IV-J, page 23 (December, 1978) or JP-B-49-25500, JP-B-43-4933, JP-A-59-19032 and JP-A-59-192242.

When the sensitizing dyes are added to the silver halide emulsions, they may be directly dispersed in the emulsions, or may be dissolved in single or mixed solvents of water, methanol, ethanol, propanol, acetone, methyl cellosolve, 2,2,3,3-tetrafluoropropanol, 2,2,2-trifluoroethanol, 3-methoxy-1-propanol, 3-methoxy-1-butanol, 1-methoxy-2-propanol and N,N-dimethylformamide, and added to the emulsions as solutions.

Further, methods which can be used in the present invention include a method of dissolving a dye in a volatile organic solvent, dispersing the resulting solution into water or a hydrophilic colloid, and adding the resulting dispersion to an emulsion, as described in U.S. Patent 3,469,987; a method of dissolving a dye in an acid, and adding the resulting solution to an emulsion, or dissolving a dye in water in the presence of an acid or a base, and adding the resulting aqueous solution to an emulsion, as described in JP-B-44-23389, JP-B-44-27555 and JP-B-57-22091; a method of dissolving or dispersing a dye into water in the presence of a surfactant, and adding the resulting aqueous solution or colloidal dispersion to an emulsion, as described in U.S. Patents 3,822,135 and 4,006,025; a method of directly dispersing a dye into a hydrophilic colloid, and adding the resulting dispersion to an emulsion, as described in JP-A-53-102733 and JP-A-58-105141; and a method of dissolving a dye by the use of a red-shifting compound, and adding the resulting solution

to an emulsion, as described in JP-A-51-74624. Further, ultrasonic waves can also be applied to the solution.

The sensitizing dyes used in the present invention may be added at any stages of the preparation of the silver halide emulsions which have hitherto been accepted to be useful. For example, they may be added during a silver halide grain formation stage and/or before desalting, during desalting stage and/or from after desalting to before the start of chemical ripening, as described in U.S. Patents 2,735,766, 3,628,960, 4,183,756 and 4,225,666, JP-A-58-184142 and JP-A-60-196749, or at any time and stage before the coating of emulsions, such as immediately before or during chemical ripening, or from after chemical ripening to the coating of the emulsions, as described in JP-A-58-113920. Furthermore, as disclosed in U.S. Patent 4,225,666 and JP-A-58-7629, the same compound may be singly added, or in combination with a compound having a different structure, divided, for example, into during a grain formation stage and during or after chemical ripening, or before or during chemical ripening and after chemical ripening. The kinds of compounds added in parts and combinations thereof may be changed.

In the present invention, the sensitizing dye may be used in a desired amount depending on performances such as sensitivity and fog. However, it is used preferably in an amount of $10^{-6}$ mol to 1 mol, and more preferably in an amount of $10^{-4}$ mol to $10^{-1}$ mol, per mol of silver halide of the light-sensitive layer.

In the present invention, the silver halide emulsion and/or the organic silver salt can be protected against formation of additional fog and stabilized against a reduction in sensitivity during storage by addition of an antifoggant, a stabilizer and a stabilizer precursor. Examples of the antifoggants, stabilizers and stabilizer precursors, which may be either alone or in combination, include thiazonium salts described in U.S. Patents 2,131,038 and 2,694,716, azaindenes described in U.S. Patents 2,886,437 and 2,444,605, mercury salts described in U.S. Patent 2,728,663, urazole described in U.S. Patent 3,287,135, sulfocatechol described in U.S. Patent 3,235,652, oximes, nitrones and nitroindazole described in GB-623,448, multivalent metal salts described in U.S. Patent 2,839,405, thiuronium salts described in U.S. Patent 3,220,839, palladium, platinum and gold salts described in U.S. Patents 2,566,263 and 2,597,915, halogen-substituted organic compounds described in U.S. Patents 4,108,665 and 4,442,202, triazine described in U.S. Patents 4,128,557, 4,137,079, 4,138,365 and 4,459,350, and phosphorus compounds described in U.S. Patent 4,411,985.

The antifoggants preferably used in the present invention are organic halides, and examples thereof include compounds as disclosed in JP-A-50-119624, JP-A-50-120328, JP-A-51-121332, JP-A-54-58022, JP-A-56-70543, JP-A-56-99335, JP-A-59-90842, JP-A-61-129642, JP-A-62-129845, JP-A-6-208191, JP-A-7-5621, JP-A-7-2781, JP-A-8-15809, U.S. Patents 5,340,712, 5,369,000 and 5,464,737.

The antifoggants used in the present invention may be added in any forms such as solutions, powders or fine solid particle dispersions. The fine solid particles are dispersed by well-known finely dividing means (for example, ball mills, vibrating ball mills, sand mills, colloid mills, jet mills and roller mills). Further, dispersing aids may be used in dispersing the fine solid particles.

Although not necessary for the practice of the present invention, it is sometimes advantageous to add mercury (II) salts as the antifoggants to the emulsions. The mercury (II) salts preferred for this purpose are mercury acetate and mercury bromide. The amount of mercury used in the present invention is preferably $1\times10^{-9}$ mol to $1\times10^{-3}$ mol, and more preferably $1\times10^{-9}$ mol to $10^{-4}$ mol, per mol of silver coated.

The photo thermographic light-sensitive materials of the present invention may also contain benzoic acid derivatives for obtaining high sensitivity and preventing fog. Any benzoic acid derivatives may be used in the present invention. However, preferred examples thereof include compounds described in U.S. Patents 4,784,939 and 4,152,160, JP-A-9-329865, JP-A-9-329864 and JP-A-9-281637. The benzoic acid derivatives may be added to any sites of the light-sensitive materials. However, they are preferably added to layers on faces having the light-sensitive layers, and more preferably to organic silver salt-containing layers. The benzoic acid derivatives used in the present invention may be added at any stages of the preparation of the coating solutions. When added to the organic silver salt-containing layers, they may be added at any stages from the preparation of the organic silver salts to the preparation of the coating solutions, preferably from after the preparation of the organic silver salts to immediately before coating. The benzoic acid derivatives used in the present invention may be added to the coating solutions in any forms such as solutions, powders or fine solid particle dispersions. Further, they may be added as solutions in which they are mixed with other additives such as sensitizing dyes, reducing agents and toning agents. The benzoic acid derivatives used in the present invention may be added in any amount, but preferably in an amount of $1\times10^{-6}$ mol to 2 mol, more preferably $1\times10^{-3}$ mol to 0.5 mol, per mol of silver.

In the present invention, the light-sensitive materials may contain mercapto compounds, disulfide compounds or thione compounds for inhibiting or accelerating development, improving the spectral sensitizing efficiency and improving keeping quality before and after development.

When the mercapto compounds are used in the present invention, they may have any structure. However, a compound represented by Ar-SM or Ar-S-S-Ar (wherein M represents a hydrogen atom or an alkali metal atom, and Ar represents an aromatic or condensed aromatic ring having at least one of nitrogen, sulfur, oxygen, selenium and tellurium) is preferably used. An aromatic heterocycle is preferably benzimidazole, naphthimidazole, benzothiazole, naphthothiazole, benzoxazole, naphthoxazole, benzoselenazole, benzotellurazole, imidazole, oxazole, pyrazole, triazole,

thiadiazole, tetrazole, triazine, pyrimidine, pyridazine, pyrazine, pyridine, purine, quinoline or quinazoline. This aromatic heterocycle may have a substituent group, for example, selected from the group consisting of halogen (for example, Br and Cl), hydroxyl, amino, carboxyl, alkyl (for example, alkyl having at least one carbon atom, preferably 1 to 4 carbon atoms) and alkoxyl (for example, alkoxyl having at least one carbon atom, preferably 1 to 4 carbon atoms). Mercapto-substituted aromatic heterocyclic compounds include, but are not limited to, 2-mercaptobenzimidazole, 2-mercaptobenzoxazole, 2-mercaptobenzothiazole, 2-mercapto-5-methylbenzimidazole, 6-ethoxy-2-mercaptobenzothiazole, 2,2'-dithiobis(benzothiazole), 3-mercapto-1,2,4-triazole, 4,5-diphenyl-2-imidazolethiol, 2-mercaptoimidazole, 1-ethyl-2-mercaptobenzimidazole, 2-mercaptoquinoline, 8-mercaptopurine, 2-mercapto-4(3H)-quinazoline, 7-trifluoromethyl-4-quinoline-thiol, 2,3,5,6-tetrachloro-4-pyridinethiol, 4-amino-6-hydroxy-2-mercaptopyrimidine monohydrate, 2-amino-5-mercapto-1,3,4-thiadiazole, 3-amino-5-mercapto-1,2,4-triazole, 4-hydroxy-2-mercaptopyrimidine, 2-mercaptopyrimidine, 4,6-diamino-2-mercaptopyrimidine, 2-mercapto-4-methylpyrimidine hydrochloride, 3-mercapto-5-phenyl-1,2,4-triazole and 2-mercapto-4-phenyloxazole.

The amount of the mercapto compound added is preferably 0.001 mol to 1.0 mol, and more preferably 0.01 mol to 0.3 mol, per mol of silver.

In the light-sensitive layers in the present invention, polyhydric alcohols (for example, glycerin and diols described in U.S. Patent 2,960,404), fatty acids or esters described in U.S. Patents 2,588,765 and 3,121,060, and silicone resins described in GB-955,061 can be used as plasticizers and lubricants.

In the present invention, super contrast-increasing agent can be used for formation of super contrast images. For example, hydrazine derivatives described in U.S. Patents 5,464,738, 5,496,695, 6,512,411 and 5,536,622, JP-A-10-10672, JP-A-10-10672, JP-A-10-62898, JP-A-10-31282, JP-A-9-319048, JP-A-9-304870 and JP-A-9-304872, quaternary nitrogen atom-containing compounds described in JP-A-9-274274, and acrylonitrile compounds described in U.S. Patent 5,545,515 can be used. Specific examples of the compounds include compounds 1 to 10 described in U.S. Patent 5,464,738, H-1 to H-28 described in U.S. Patent 5,496,695, I-1 to I-86 described in JP-A-10-10672, H-1 to H-62 described in JP-A-10-62898, 1-1 to 1-21 described in JP-A-10-31282, 1 to 50 described in JP-A-9-304870, 1 to 40 described in JP-A-9-304872, P-1 to P-26 and T-1 to T-18 described in JP-A-9-274274 and CN-1 to CN-13 described in U.S. Patent 5,545,515.

In the present invention, for formation of super contrast images, super contrast increasing accelerators can be used in combination with the above-mentioned super contrast increasing agents. For example, amine compounds described in U.S. Patent 5,545,505, specifically, AM-1 to AM-5, hydroxamic acids described in U.S. Patent 5,545,507, specifically, HA-1 to HA-11, acrylonitrile compounds described in U.S. Patent 5,545,507, specifically, CN-1 to CN-13, hydrazine compounds described in U.S. Patent 5,558,983, specifically, CA-1 to CA-6, onium salts described in JP-9-297368, specifically, A-1 to A-42, B-1 to B-27 and C-1 to C-14 can be used.

Synthesis and addition of these super contrast increasing agents and super contrast increasing accelerators can be performed according to the descriptions of the above-mentioned cited patents, and the amounts of the agents and the accelerators added can be determined according to them.

The image formation material in the present invention may be provided with a surface protection layer for preventing adhesion of the image formation layer.

Although binders for the surface protection layers used in the present invention may be any polymers, it is preferred that they contain carboxylic acid residue-containing polymers in an amount of 100 $mg/m^2$ to 5 $g/m^2$. The carboxylic acid residue-containing polymers as used herein include natural polymers (gelatin and alginic acid), modified natural polymers (carboxymethyl cellulose and phthalated gelatin) and synthetic polymers (polymethacrylates, polyacrylates, polyalkyl methacrylate/acrylate copolymers and polystyrene/polymethacrylate copolymers). The amount of carboxyl residues contained in said polymer is preferably $1 \times 10^{-2}$ mol to 1.4 mol per 100 g of polymer. Further, the carboxylic acid residues may form salts with alkali metal ions, alkaline earth metal ions or organic cations.

Any adhesion preventing materials may be used for the surface protection layers in the present invention. Examples of adhesion preventing materials include wax, silica particles, styrene-containing elastomeric block copolymers (such as styrene-butadiene-styrene and styrene-isoprene-styrene), cellulose acetate, cellulose acetate butylate, cellulose propionate and mixtures thereof. Further, crosslinking agents for crosslinking and surfactants for improving coating properties may be added to the surface protection layers.

In the image formation layers or the protection layers for the image formation layers used in the present invention, photographic elements containing light absorption materials and filter dyes as described in U.S. Patents 3,253,921, 2,274,782, 2,527,583 and 2,956,879 can be used. Furthermore, the dyes can be mordanted as described, for example, in U.S. Patent 3,282,699. The filter dyes are used in such an amount as to give an absorbance at an exposure wavelength of preferably 0.1 to 3.0, more preferably 0.2 to 1.5.

The image formation layers or the protection layers for the image formation layers used in the present invention can contain delustering agents such as starch, titanium dioxide, zinc oxide, silica and polymer beads containing beads of kinds described in U.S. Patents 2,992,101 and 2,701,245. The matte degree of emulsion surfaces may be any, as long as no white-spot unevenness occurs. However, the Beck smoothness is preferably from 50 seconds to 10,000 seconds, and particularly preferably from 80 seconds to 10,000 seconds.

The preparation temperature of the coating solutions for the image formation layers used in the present invention is preferably from 30°C to 65°C, and more preferably from 35°C to less than 60°C. Further, the temperature of the coating solutions for the image formation layers immediately after addition of the polymer latexes is preferably maintained at a temperature of 30°C to 65°C. Furthermore, it is preferred that the reducing agents and the organic silver salts are mixed before addition of the latexes.

The organic silver salt-containing fluids or the coating solutions for the image formation layers used in the present invention are preferably so-called thixotropic fluids. The thixotropy means the property that the viscosity decreases with an increase in the shear rate. Although any instruments may be used for measurement of the viscosity in the present invention, an RFS fluid spectrometer (manufactured by Rheometric Far East Co.) is preferably used and measurements are made at 25°C. Here, for the organic silver salt-containing fluids or the coating solutions for the image formation layers used in the present invention, the viscosity at a shear rate of 0.1 $S^{-1}$ is preferably from 400 mPa • s to 100,000 mPa • s, and more preferably from 500 mPa • s to 20,000 mPa • s. Further, the viscosity at a shear rate of 1,000 $S^{-1}$ is preferably from 1 mPa • s to 200 mPa • s, and more preferably from 5 mPa • s to 80 mPa • s.

Various kinds of systems exhibiting the thixotropy are known, and described in Course Rheology, edited by Kobunshi Kankokai, and Muroi and Morino, Polymer Latexes (published by Kobunshi Kankokai. For allowing fluids to exhibit the thixotropy, they are required to contain many fine solid particles. Further, for enhancing the thixotropy, it is effective to allow thickening linear polymers to be contained, to increase the aspect ratio by the anisotropic form of the fine solid particles contained, and to use alkali thickening agents and surfactants.

The photothermographic light-sensitive emulsion or emulsions of the present invention are applied onto a support as one or more layers. The one layer is required to contain the organic silver salt, the silver halide, a developing agent and the binder, and optional additional materials such as the toning agent, a coating aid and other auxiliary agents. For the two layer structure, a first emulsion layer (usually, a layer adjacent to the substrate) is required to contain the organic silver salt and the silver halide, and a second layer or both layers must contain some other components. However, a single emulsion layer containing all components and the two-layer structure comprising a protection top coat is also conceivable. The structure of a photothermographic multicolor-sensitive photographic material may contain a combination of these two layers for each color, or all components in a single layer as described in U.S. Patent 4,708,928. In the case of a photothermographic multi-dye multicolor-sensitive photographic material, respective emulsion layers are generally kept distinguished from each other by using a functional or nonfunctional barrier layer between respective light-sensitive layers, as described in U.S. Patent 4,460,681.

The light-sensitive layers used in the present invention can contain various kinds of dyes and pigments from the viewpoint of improvement in a color tone and prevention of irradiation. Although any dyes and pigments may be used in the light-sensitive layers of the present invention, for example, dyes and pigments described in Color Index can be used. Specific examples thereof include organic dyes such as pyrazoloazole dyes, anthraquinone dyes, azo dyes, azomethine dyes, oxonol dyes, carbocyanine dyes, styryl dyes, triphenylmethane dyes, indoaniline dyes and indophenol dyes, organic pigments including azo pigments, polycyclic pigments (phthalocyanine pigments and anthraquinone pigments), dyeing lake pigments and azine pigments, and inorganic pigments. Preferred examples of the dyes used in the present invention include anthraquinone dyes (for example, compounds 1 to 9 described in JP-A-5-341441, and compounds 3-6 to 3-18 and 3-23 to 3-38 described in JP-A-5-165147), azomethine dyes (compounds 17 to 47 described in JP-A-5-341441), indoaniline dyes (for example, compounds 11 to 19 described in JP-A-5-289227, compound 47 described in JP-A-5-341441, and compounds 2-10 and 2-11 described in JP-A-5-165147), and azo dyes (compounds 10 to 16 described in JP-A-5-341441). Preferred examples of the pigments include indanthrone pigments of the anthraquinone series (C.I. Pigment Blue 60), phthalocyanine pigments (copper phthalocyanine such as C.I. Pigment Blue 15 and non-metal phthalocyanine such as C.I. Pigment Blue 16), triarylcarbonyl pigments of the dyeing lake pigment series, indigo and inorganic pigments (ultramarine blue and cobalt blue). These dyes and pigments may be added in any forms such as solutions, emulsions and fine solid particle dispersions, or in state where the dyes are mordanted with polymer mordants. Although the amount of these compounds used is determined depending upon the desired amount to be absorbed, it is generally preferred that they are used in an amount of 1 μg to 1 g per $m^2$. For adjusting reddish colors, dioxane pigments, quinacridone pigments or diketopyrrolo-pyrrole may be used in combination.

In the present invention, an antihalation layer can be provided on the side far away from a light source with respect to the light-sensitive layer. The antihalation layer preferably has a maximum absorption of 0.3 to 2 in a desired wavelength range, more preferably has an absorption at an exposure wavelength of 0.5 to 2. Further, the antihalation layer preferably has an absorption of 0.001 to less than 0.5 in a visible region after treatment, and more preferably has an optical density of 0.001 to less than 0.3.

When an antihalation dye is used in the present invention, it may be any compound, as long as it has a desired absorption in the wavelength range, is sufficiently low in absorption in the visible region after treatment, and gives a shape of a preferred absorption spectrum of the above-mentioned antihalation layer. Examples thereof include but are

not limited to the following compounds. Examples of independent dyes include compounds described in JP-A-59-56458, JP-A-2-216140, and JP-A-7-13295, JP-A-7-11432, U.S. Patent 5,380,635, JP-A-2-68539, page 13, lower left column, line 1 to pag 14, lower left column, line 9 and JP-A-3-24539, page 14, lower left column to page 16, lower right column, and examples of dyes whose color disappears by treatment include compounds described in JP-A-52-139136, JP-A-53-132334, JP-A-56-501480, JP-A-57-16060, JP-A-57-68831, JP-A-57-101835, JP-A-59-182436, JP-A-7-36145, JP-A-7-199409, JP-B-48-33692, JP-B-50-16648, JP-B-2-41734, U.S. Patents 4,088,497, 4,283,487, 4,548,896 and 5,187,049.

It is preferred that the photo thermographic light-sensitive material of the present invention is a so-called single light-sensitive material having at least one silver halide emulsion-containing light-sensitive layer on one side of the support and a back layer on the other side.

In respect to the single light-sensitive material of the present invention, a matting agent may be added for improving the transferring properties. The matting agent is generally fine particles of a water-insoluble organic or inorganic compound, and any one can be used. For example, matting agents well known in the art such as organic matting agents described in U.S. Patents 1,939,213, 2,701,245, 2,322,037, 3,262,782, 3,539,344 and 3,767,448 and inorganic matting agents described in U.S. Patents 1,260,772, 2,192,241, 3,257,206, 3,370,951, 3,523,022 and 3,769,020 can be used. Specific examples of the organic compounds which can be used as the matting agents include water-dispersible vinyl polymers such as polymethyl acrylate, polymethyl methacrylate, polyacrylonitrile, acrylonitrile-$\alpha$-methylstyrene copolymers, polystyrene, styrene-divinylbenzene copolymers, polyvinyl acetate, polyethylene carbonate and polytetrafluoroethylene, cellulose derivatives such as methyl cellulose, cellulose acetate and cellulose acetate propionate, and starch derivatives such as carboxystarch, carboxynitrophenyl starch and urea-formaldehyde-starch reaction products. Gelatin hardened with a well-known hardening agent and hardened gelatin formed into fine capsule hollow granules by coacervate hardening can also be preferably used. Preferred examples of the inorganic compounds include silicon dioxide, titanium dioxide, magnesium dioxide, aluminum oxide, barium sulfate, calcium carbonate, silver chloride and silver bromide desensitized by well-known methods, glass and diatomaceous earth. The above-mentioned matting agents can be used as mixtures with different kinds of substances if necessary. There is no particular limitation on the size and shape of the matting agents, and ones having any particle size can be used. In the practice of the present invention, ones having a particle size of 0.1 $\mu$m to 30 $\mu$m are preferably used. The particle size distribution of the matting agents may be either narrow or wide. On the other hand, the matting agents significantly influence the haze and surface luster of the light-sensitive materials. It is therefore preferred that the size, shape and particle size distribution are adjusted to required conditions in the preparation of the matting agents or by mixing the plurality of matting agents.

In the present invention, as the matte degree of the back layers, the Beck smoothness is preferably from 10 seconds to 250 seconds, and more preferably from 50 seconds to 180 seconds.

In the present invention, the matting agent is preferably contained in the outermost surface layer, a layer which functions as the outermost layer, or a layer close to the outer surface, of the light-sensitive material, and preferably contained in a layer which functions as the so-called protection layer.

In the present invention, suitable binders for the back layers are transparent or translucent, and generally colorless. They are natural and synthetic resins (polymers and copolymers) and other film forming media, and examples thereof include gelatin, gum arabic, polyvinyl alcohol, hydroxyethyl cellulose, cellulose acetate, cellulose acetate butylate, polyvinylpyrrolidone, casein, starch, polyacrylic acid, polymethyl methacrylate, polyvinyl chloride, polymethacrylic acid, copoly(styrene-maleic anhydride), copoly(styrene-acrylonitrile), copoly(styrene-butadiene), polyvinyl acetals (for example, polyvinyl formal and polyvinyl butyral), polyesters, polyurethanes, phenoxy resins, polyvinylidene chloride, polyepoxides, polycarbonates, polyvinyl acetate, cellulose esters and polyamides. The binders may be formed from aqueous solutions, organic solvent solutions or emulsions by coating.

In the present invention, the maximum absorption in a desired wavelength range of the back layer is preferably 0.3 to 2, and more preferably 0.5 to 2, and the absorption in the visible region after treatment is 0.001 to less than 0.5. Further, a layer having an optical density of 0.001 to less than 0.3 is more preferred. Examples of antihalation dyes used in the back layers are the same as described above for the antihalation layers.

A backside resistive heating layer disclosed in U.S. Patents 4,460,681 and 4,374,921 can also be used in the photo thermographic light-sensitive photographic image system of the present invention.

A hardener may be used in each layer of the light-sensitive layer, the protection layer and the back layer of the present invention. Examples of the hardeners are described in T. H. James, THE THEORY OF THE PHOTOGRAPHIC PROCESS FOURTH EDITION, pages 77 to 87, published by Macmillan Publishing Co., Inc. (1977), and multivalent metal ions described in ibid., page 78, polyisocyanates described in U.S. Patent 4,281,060 and JP-A-6-208193, epoxy compounds described in U.S. Patent 4,791,042 and vinylsulfone compounds described in JP-A-62-89048 are preferably used.

The hardeners are added as solutions, and the solutions are preferably added to the coating solutions for image forming layers from 180 minutes before coating to immediately before coating, preferably from 60 minutes before coating to 10 seconds before coating. However, there is no particular limitation on the mixing process and the mixing

conditions, as long as the effects of the present invention are sufficiently manifested. Specific examples of the mixing processes include a mixing process using a tank designed so that the average residence time calculated from the flow rate of the solution added and the amount of the solution supplied to a coater becomes a desired time, and a process using static mixers described in N. Harnby, M. F. Edwards and A. W. Nienow, translated by Koji Takahashi, Liquid Mixing Techniques, chapter 8, published by Nikkan Kogyo Shinbunsha (1989).

In the present invention, surfactants may be used for improving the coating property and the antistatic property. As the surfactants, any surfactants such as nonionic, anionic, cationic and fluorine surfactants may be appropriately used. Specific examples thereof include fluorine polymer surfactants described in JP-A-62-170950 and U.S. Patent 5,380,644, fluorine surfactants described in JP-A-60-244945 and JP-A-63-188135, polysiloxane surfactants described in U.S. Patent 3,885,965, and polyalkylene oxides and anionic surfactants described in JP-A-6-301140.

Examples of the solvents used in the present invention are described in New Pocket Book of Solvents (Ohm Co., 1994), but the present invention is not limited thereto. The boiling point of the solvents used in the present invention is preferably 40°C to 180°C.

Examples of the solvents used in the present invention include hexane, cyclohexane, toluene, methanol, ethanol, isopropanol, acetone, methyl ethyl ketone, ethyl acetate, 1,1,1-trichloroethane, tetrahydrofuran, triethylamine, thiophene, trifluoroethanol, perfluoropentane, xylene, n-butanol, phenol, methyl isobutyl ketone, cyclohexanone, butyl acetate, diethyl carbonate, chlorobenzene, dibutyl ether, anisole, ethylene glycol diethyl ether, N,N-dimethyl-formamide, morpholine, propanesultone, perfluorotributylamine and water.

Various supports can be coated with the photo thermographic light-sensitive emulsions used in the present invention. Typical examples of the supports include polyester films, undercoated polyester films, polyethylene terephthalate films, polyethylene naphthalate films, cellulose nitrate films, cellulose ester films, polyvinyl acetal films, polycarbonate films, related or resinous materials, glass, paper and metals. In particular, paper supports partially acetylated or coated with baryta and/or $\alpha$-olefin polymers, particularly $\alpha$-olefin polymers each having 2 to 10 carbon atoms such as polyethylene, polypropylene and ethylene-butene copolymers are typically used. Although the supports may be transparent or opaque, they are preferably transparent.

The light-sensitive materials of the present invention may have antistatic or conductive layers such as layers containing soluble salts (for example, chlorides and nitrates), metal-deposited layers, ionic polymer-containing layers described in U.S. Patents 2,861,056 and 3,206,312, and/or insoluble inorganic salt-containing layers described in U.S. Patent 3,428,451.

Methods for obtaining color images by using the photothermographic light-sensitive materials of the present invention include methods described in JP-A-7-13295, page 10, left column, line 43 to page 11, left column, line 40. Further, examples of stabilizers for color dye images are described in British Patent 1,326,889, U.S. Patents 3,432,300, 3,698,909, 3,574,627, 3,573,050, 3,764,337 and 4,042,394.

The photothermographic light-sensitive materials of the present invention may be applied by any methods. Specifically, various coating operations including extrusion coating, slide coating, curtain coating, dip coating, knife coating, flow coating and extrusion coating using a hopper described in U.S. Patent 2,681,294 are used. Extrusion coating described in Stephen F. Kistler and Petert M. Schweizer, LIQUID FILM COATING, pages 399 to 536, published by CHAPMAN & HALL (1997) or slide coating is preferably used, and slide coating is particularly preferably used. Examples of the shapes of slide coaters used in slide coating are shown in ibid., Figure 11b. 1 on page 427. Two or more layers can be formed at the same time by methods described in ibid., pages 399 to 536, U.S. Patent 2,761,791 and GB-837,095, as so desired.

The photothermographic light-sensitive materials of the present invention can contain additional layers such as dye receiving layers for receiving mobile dye images, opaqued layers used in cases where reflection printing is desired, protection top coat layers and primer layers known in the light-heat photographic art. In the light-sensitive material of the present invention, it is preferred that the use of only one sheet of the light-sensitive material permits image formation, not accompanied by another layer having functions necessary for image formation, such as an image receiving layer.

The light-sensitive materials of the present invention may be developed by any methods. However, the light-sensitive materials exposed imagewise are usually developed by elevating the temperature thereof. The developing temperature is preferably from 80°C to 250°C, and more preferably from 100°C to 140°C. The developing time is preferably from 1 second to 180 seconds, and more preferably from 10 seconds to 90 seconds.

Although the light-sensitive materials of the present invention may be exposed by any methods, laser light sources are preferably used as exposure light sources. Preferred examples of the lasers used in the present invention include gas lasers, YAG lasers, dye lasers and semiconductor lasers. Further, semiconductor lasers and second harmonic generating elements can also be used in combination.

The light-sensitive materials of the present invention tend to be low in haze at exposure and develop interference fringes. As methods for preventing the interference fringes from being developed, a method of allowing laser light to be obliquely incident upon the light-sensitive material as disclosed in JP-A-5-113548 and a method utilizing a multimode laser disclosed in WO95/31754 are known, and these methods are preferably used.

When the light-sensitive materials of the present invention are exposed, it is preferred that they are exposed so that laser light overlaps to make scanning lines invisible, as disclosed in SPIE, vol. 169, Laser Printing, pages 116 to 128 (1979), JP-A-4-51043 and WO95/31754.

The present invention will be described in more detail with reference to the following examples.

EXAMPLE 1

⟨Preparation of Support⟩

Using terephthalic acid and ethylene glycol, PET having an IV of 0.66 (measured in phenol/tetrachloroethane (having a weight ratio of 6/4) at 25°C) was obtained. This was pelletized, and dried at 130°C for 4 hours. Then, this was melted at 300°C, and extruded through a T die, followed by rapid cooling to prepare an unstretched film having such a thickness as to give a film thickness of 175 μm after heat setting.

This unoriented film was stretched lengthwise 3.3 times by use of rolls different from each other in peripheral speed, and then, stretched crosswise 4.5 times with a tenter. At this time, the temperatures were 110°C and 130°C, respectively. Then, the stretched film was heat set at 240°C for 20 seconds, and thereafter relaxed crosswise by 4% at the same temperature. Then, after portions chucked with the tenter was slit off, the knurl treatment was applied to both edges. Then, the resulting film was wound up at a tension of 4 kg/cm$^2$ to obtain a roll of the film having a thickness of 175 μm.

⟨Surface Corona Treatment⟩

Both surfaces of the support were treated with a Model 6KVA solid state corona treating device manufactured by Pillar Co. at room temperature at 20 m/min. Readings of current and voltage at this time revealed that the support was treated at 0.375 kV • A • min./m$^2$. The treatment frequency at this time was 9.6 kHz, and the gap clearance between an electrode and a dielectric roll was 1.6 mm.

⟨Preparation of Undercoated Support⟩

(Preparation of Coating Solution A for Undercoat)

One gram of fine polystyrene particles (average particle size: 0.2% μm) and 20 ml of surfactant 1 (1% by weight) were added to 200 ml of an aqueous dispersion of a polyester copolymer, Pesresin A-515GB (30%, manufactured by Takamatsu Yushi Co.), and distilled water was added thereto to bring the volume to 1000 ml, thus preparing coating solution A for undercoat.

(Preparation of Coating Solution B for Undercoat)

To 680 ml of distilled water, 200 ml of an aqueous dispersion of a styrene-butadiene copolymer (styrene/butadiene/itaconic acid = 47/50/3 (weight ratio), concentration: 30% by weight) and 0.1 g of fine polystyrene particles (average particle size: 2.5 μm) were added, and distilled water was added thereto to bring the volume to 1000 ml, thus preparing coating solution B for undercoat.

(Preparation of Coating Solution C for Undercoat)

Ten grams of inert gelatin was dissolved in 500 ml of distilled water, and 40 g of an aqueous dispersion (40% by weight) of a tin oxide-antimony oxide complex described in JP-A-61-20033 was added thereto. Then, distilled water was added thereto to bring the volume to 1000 ml, thus preparing coating solution C for undercoat.

(Preparation of Undercoated Support)

The support subjected to the above-mentioned corona discharge treatment was coated with coating solution A for undercoat with a bar coater so as to give a wet amount coated of 5 ml/m$^2$, and dried at 180°C for 5 minutes. The dried film thickness thereof was about 0.3 μm. Then, the back face thereof was subjected to the corona discharge treatment, and coated with coating solution B for undercoat with a bar coater so as to give a wet amount coated of 5 ml/m$^2$ and a dried film thickness of about 0.3 μm, followed by drying at 180°C for 5 minutes. Coating solution C for undercoat was further applied onto it with a bar coater so as to give a wet amount coated of 3 ml/m$^2$ and a dried film thickness of

about 0.03 μm, followed by drying at 180°C for 5 minutes. Thus, an undercoated support was prepared.

Fatty acid silver salts were prepared as follows:

〈Preparation of Fatty Acid Silver Salts A to N〉

In a reaction vessel, 640 ml of distilled water and 25 ml of tert-butyl alcohol were placed, and kept at a temperature shown in Table 1. Then, 206 ml of a Ag solution (an aqueous solution containing 40.35 g of silver nitrate) cooled to 8°C was constantly added for an addition time shown in Table 1 with vigorous stirring. At the time when the Ag solution was added up to the ratio shown in Table 1 to the total amount of Ag solution added, a sodium behenate solution kept at 75°C was constantly added for addition time shown in Table 1. The term "-10%" as used herein means that 10% of the sodium behenate solution was previously added. After the addition of the sodium behenate solution was completed, ripening was conducted for 20 minutes. Then, the temperature was lowered to a temperature shown in Table 1. Aqueous dispersions of fatty acid silver salts A to N having shapes and characteristics shown in Table 2 were prepared by changing the reaction temperature and the addition time. The equivalent-sphere diameter, long side/short side, aspect ratio (A. R.) and particle thickness in Table 2 are each an average value.

The sodium behenate solution used for addition was prepared in the following manner. Namely, 87.7 g of behenic acid (trade name: Edenor C22-85R) manufactured by Henckel Co., 418 ml of distilled water, 120 ml of tert-butanol and 9.85 g of 100% NaOH pellets were mixed, and stirred at 75°C for 1 hour to conduct the reaction.

TABLE 1

| Fatty Acid Silver Salt | Reaction Temp. (°C) | Addition Time of Silver Nitrate (min.) | Previous Addition of Silver (%) | Addition Time of Sodium Behenate | Decrease in Temp. (°C) |
|---|---|---|---|---|---|
| A | 30 | 60 | 10 | 62 min. | 25 |
| B | 30 | 220 | 10 | 227 min. | 25 |
| C | 30 | 350 | 10 | 361 min. | 25 |
| D | 30 | 20 | 10 | 21 min. | 25 |
| E | 30 | 5 | 10 | 5 min. and 9 sec. | 25 |
| F | 30 | 60 | 50 | 62 min. | 25 |
| G | 30 | 60 | 70 | 62 min. | 25 |
| H | 30 | 60 | 90 | 62 min. | 25 |
| I | 30 | 60 | 95 | 62 min. | 25 |
| J | 75 | 60 | 10 | 62 min. | 25 |
| K | 10 | 35 | 10 | 36 min. | 15 |
| L | 15 | 50 | 10 | 52 min. | 20 |
| M | 35 | 85 | -10 | 88 min. | 25 |
| N | 45 | 100 | -10 | 103 min. | 25 |

TABLE 2

| Fatty Acid Silver Salt Particle | Equivalent-Sphere Diameter (μm) | Long Side/Short side | A. R. | Particle Thickness (μm) | Coefficient of Variation (%) | Ratio of Simultaneous Addition (%) |
|---|---|---|---|---|---|---|
| A (Invention) | 0.51 | 1.5 | 5.1 | 0.14 | 15 | 90 |
| B (Invention) | 0.12 | 1.4 | 5 | 0.03 | 14.3 | 90 |
| C (Comparison) | 0.03 | 1.5 | 2 | 0.01 | 15.2 | 90 |

TABLE 2 (continued)

| Fatty Acid Silver Salt Particle | Equivalent-Sphere Diameter (μm) | Long Side/Short side | A. R. | Particle Thickness (μm) | Coefficient of Variation (%) | Ratio of Simultaneous Addition (%) |
|---|---|---|---|---|---|---|
| D (Invention) | 0.79 | 1.5 | 4.9 | 0.18 | 14.9 | 90 |
| E (Comparison) | 1.05 | 1.5 | 5 | 0.29 | 16 | 90 |
| F (Invention) | 0.49 | 1.5 | 5 | 0.14 | 15.3 | 50 |
| G (Invention) | 0.5 | 1.5 | 5 | 0.14 | 14.8 | 30 |
| H (Invention) | 0.5 | 1.4 | 5.1 | 0.14 | 15.1 | 10 |
| I (Comparison) | 0.48 | 1.5 | 5 | 0.13 | 17 | 5 |
| J (Comparison) | 0.5 | 12.5 | 5 | 0.14 | 22 | 90 |
| K (Comparison) | 0.51 | 1.4 | 1 | 0.4 | 7 | 90 |
| L (Comparison) | 0.49 | 1.5 | 2 | 0.25 | 12 | 90 |
| M (Invention) | 0.49 | 1.5 | 5 | 0.14 | 34 | 93 |
| N (Invention) | 0.5 | 1.4 | 4.9 | 0.14 | 40 | 93 |

Then, solid matter was filtered by suction filtration, and washed with water until a filtrate showed a conductivity of 30 μS/cm. Thus, fatty acid silver salts A to N were obtained.

Fatty acid silver salts A to N were redispersed in the following manner to prepare redispersed products A to N of the fatty acid silver salts.

〈Preparation of Redispersed Products A to N of Fatty Acid Silver Salts〉

To a wet cake corresponding to 100 g of dried solid matter of each of fatty acid silver salts A to N, 7.4 g of polyvinyl alcohol (PVA-205, manufactured by Kuraray Co., Ltd.) and water were added to make the total weight 385 g, and the resulting mixture was preliminarily dispersed with a homomixer.

Then, the original fluid preliminarily dispersed was treated three times with a dispersing device (trade name: Microfluidizer M-110S-EH, manufactured by Microfluidex International Corporation, using a G10Z interaction chamber), adjusting its pressure to 1750 $kg/cm^2$. Thus, each of redispersed products A to F of the fatty acid silver salts was obtained. The particle size of redispersed products A to F of the fatty acid silver salts was the same as that shown in Table 2. For the cooling operation in dispersion, coiled heat exchangers were each mounted in front of and behind the interaction chamber, and the temperature of a refrigerant was controlled thereby to set the dispersing temperature to a desired temperature.

〈Preparation of 25% Dispersion of Reducing Agent〉

To 80 g of 1,1-bis(2-hydroxy-3,5-dimethylphenyl)-3,5,5-trimethylhexane and 64 g of a 20% aqueous solution of modified polyvinyl alcohol (Poval MP203, manufactured by Kuraray Co., Ltd.), 176 g of water was added, and sufficiently mixed to prepare a slurry. The slurry was placed in a vessel together with 800 g of zirconia beads having an average diameter of 0.5 mm, and dispersed with a dispersing device (a 1/4G sand grinder mill, manufactured by Imex Co.) for 5 hours to obtain a reducing agent dispersion. Reducing agent particles contained in the reducing agent dispersion thus obtained had an average particle size of 0.72 μm.

〈Preparation of 20% Dispersion of Mercapto Compound〉

To 64 g of 3-mercapto-4-phenyl-5-heptyl-1,2,4-triazole and 32 g of a 20% aqueous solution of modified polyvinyl alcohol (Poval MP203, manufactured by Kuraray Co., Ltd.), 224 g of water was added, and sufficiently mixed to prepare a slurry. The slurry was placed in a vessel together with 800 g of zirconia beads having an average diameter of 0.5 mm, and dispersed with a dispersing device (a 1/4G sand grinder mill, manufactured by Imex Co.) for 10 hours to obtain a mercapto dispersion. Mercapto compound particles contained in the mercapto compound dispersion thus obtained had an average particle size of 0.67 μm.

〈Preparation of 30% Dispersion of Organic Polyhalogen Compound〉

To 48 g of tribromomethylphenylsulfone, 48 g of 3-tribromomethylsulfonyl-4-phenyl-5-tridecyl-1,2,4-triazole and 48 g of a 20% aqueous solution of modified polyvinyl alcohol (Poval MP203, manufactured by Kuraray Co., Ltd.), 224 g of water was added, and sufficiently mixed to prepare a slurry. The slurry was placed in a vessel together with 800 g of zirconia beads having an average diameter of 0.5 mm, and dispersed with a dispersing device (a 1/4G sand grinder mill, manufactured by Imex Co.) for 5 hours to obtain an organic polyhalogen compound dispersion. Polyhalogen compound particles contained in the poyhalogen compound dispersion thus obtained had an average particle size of 0.74 μm.

〈Preparation of Solution of Phthalazine Compound in Methanol〉

In 100 ml of methanol, 26 g of 6-isopropylphthalazine was dissolved, and the resulting solution was used.

〈Preparation of 20% Dispersion of Pigment〉

To 64 g of C.I. Pigment Blue 60 and 6.4 g of Demol N manufactured by Kao Corp., 250 g of water was added, and sufficiently mixed to prepare a slurry. The slurry was placed in a vessel together with 800 g of zirconia beads having an average diameter of 0.5 mm, and dispersed with a dispersing device (a 1/4G sand grinder mill, manufactured by Imex Co.) for 25 hours to obtain a pigment dispersion. Pigment particles contained in the pigment dispersion thus obtained had an average particle size of 0.21 μm.

〈Preparation of Silver Halide Grains 1〉

To 1421 ml of distilled water, 6.7 ml of 1 wt% potassium bromide solution was added, and 8.2 ml of 1 N nitric acid and 21.8 g of gelatin phthalate were further added thereto. The resulting solution was maintained at 35°C in a titanium-coated stainless steel reaction pot with stirring. On the other hand, solution a1 was prepared by diluting 37.04 g of silver nitrate with distilled water to a volume of 159 ml, and solution b1 was prepared by diluting 32.6 g of potassium bromide with distilled water to a volume of 200 ml. Solution a1 was wholly added by the controlled double jet method at a constant flow rate for 1 minute while maintaining the pAg at 8.1 (solution b1 was added by the controlled double jet method.). Then, 30 ml of a 3.5% aqueous solution of hydrogen peroxide was added, and 36 ml of a 3 wt% aqueous solution of benzimidazole was further added. Thereafter, solution a2 was prepared by diluting solution a1 with distilled water to 317.5 ml, and solution b2 was prepared by dissolving dipotassium iridate hexachloride in solution b1 so as to finally give $1\times10^{-4}$ mol per mol of silver, and diluting it with water to 400 cc, twice the volume of solution b1. Solution a2 was wholly added by the controlled double jet method at a constant flow rate for 10 minutes while maintaining the pAg at 8.1 (solution b2 was added by the controlled double jet method.). Then, 50 ml of a 0.5% solution of 2-mercapto-5-methylbenzimidazole in methanol was added, and after the pAg was increased to 7.5 with silver nitrate, the pH was adjusted to 3.8 using 1 N sulfuric acid. Then, stirring was stopped, and sedimentation/desalting/ washing steps were carried out. Then, 3.5 g of deionized gelatin was added and 1 N sodium hydroxide was added to adjust the pH and the pAg to 6.0 and 8.2, respectively, to prepare a silver halide dispersion.

Grains in the resulting silver halide emulsion were pure silver bromide grains having an average equivalent-sphere diameter of 0.031 μm and a coefficient of variation of equivalent-sphere diameters of 11%. The grain size was determined from an average of 1000 grains using an electron microscope. The (100) face ratio of the grains determined by the Kubelka-Munk method was 85%.

The temperature of the emulsion was elevated to 50°C with stirring, and 5 ml of a 0.5 wt% solution of N,N'-dihydroxy-N",N"-diethylmelamine in methanol and 5 ml of a 3.5 wt% solution of phenoxyethanol in methanol were added. After one minute, sodium benzenethiosulfonate was added in an amount of $3\times10^{-5}$ mol per mol of silver, and after further 2 minutes, a solid dispersion (an aqueous solution of gelatin) of spectral sensitizing dye 1 was added in an amount of $5\times10^{-3}$ mol per mol of silver. After still further 2 minutes, a tellurium compound was added in an amount of $5\times10^{-5}$ mol per mol of silver, followed by ripening for 50 minutes. Just before the ripening was completed, 2-mercapto-5-methylbenzimidazole was added in an amount of $1\times10^{-3}$ mol per mol of silver, and the temperature was lowered to terminate the chemical ripening, thereby preparing silver halide grains 1.

〈Preparation of Silver Halide Grains 2〉

In 700 ml of water, 22 g of phthalated gelatin and 30 mg of potassium bromide were dissolved, and the pH was adjusted to 5.0 at a temperature of 35°C. Thereafter, 159 ml of an aqueous solution containing 18.6 g of silver nitrate and 0.9 g of ammonium nitrate, and an aqueous solution containing potassium bromide and potassium iodide at a

molar ratio of 92:8 were added thereto by the controlled double jet method for 10 minutes while maintaining the pAg at 7.7. Then, 476 ml of an aqueous solution containing 55.4 g of silver nitrate and 2 g of ammonium nitrate, and an aqueous solution containing $1\times10^{-5}$ mol/liter of dipotassium iridate hexachloride and 1 mol/liter of potassium bromide were added thereto by the controlled double jet method for 30 minutes while maintaining the pAg at 7.7. Thereafter, 1 g of 4-hydroxy-6-methyl-1,3,3a,7-tetraazaindene was added, and the pH was lowered to perform coagulating sedimentation, followed by desalting. Then, 0.1 g of phenoxyethanol was added to adjust the solution to pH 5.9 and pAg 8.2 to complete the preparation of silver iodobromide grains (cubic grains having a core iodine content of 8 mol%. an average content of 2 mol%, an average size of 0.05 μm, a coefficient of variation of projected area diameters of 8% and a (100) face ratio of 88%).

The temperature of the silver halide grains thus obtained was elevated to 60°C, and 85 μmol of sodium thiosulfate, $1.1\times10^{-5}$ mol of 2,3,4,5,6-pentafluorophenyl-diphenylphosphine selenide, $1.5\times10^{-5}$ mol of a tellurium compound, $3.5\times10^{-8}$ mol of chloroauric acid and $2.7\times10^{-4}$ mol of thiocyanic acid, per mol of silver, were added thereto, followed by ripening for 120 minutes. After rapid cooling to 40°C, $1\times10^{-4}$ mol of spectral sensitizing dye 1 and $5\times10^{-4}$ mol of 2-mercapto-5-methylbenzimidazole were added, followed by rapid cooling to 30°C to obtain silver halide grains 2.

〈Preparation of Coating Solutions for Emulsion Layers〉

(Coating Solutions A to N for Emulsion Layers)

Each of dispersions A to N of the fatty acid silver salts obtained above (103 g) was mixed with 5 g of a 20% aqueous solution of polyvinyl alcohol (PVA-205, manufactured by Kuraray Co., Ltd.), and the temperature of the mixture was maintained at 40°C. The above-mentioned 25 wt% dispersion of reducing agent (23.2 g), 1.2 g of the 20 wt% aqueous dispersion of pigment C.I. Pigment Blue 60, 10.7 g of the 30% dispersion of the organic polyhalide and 3.1 g of the 20 wt% dispersion of the mercapto compound were added thereto. Then, 106 g of a 40 wt% SBR latex purified by ultrafiltration (UF) and maintained at 40°C was added, and well stirred, followed by addition of 6 ml of a solution of the phthalazine compound in methanol to obtain each of fatty acid silver salt-containing solutions A to F. Further, 5 g of silver halide grains 1 and 5 g of silver halide grains 2 were previously well mixed with each other, and the resulting mixture was mixed with each of fatty acid silver salt-containing solutions A to N just before coating with a static mixer to prepare each of coating solution A to N for emulsion layers. The solution was supplied to a coating die as such so as to give an amount of silver coated of 1.4 g/m$^2$.

The viscosity of the coating solutions for emulsion layers was measured with a B type viscometer (No. 1 rotor was used) of Tokyo Keiki Co., Ltd., and was 85 mPa•s at 40°C.

The viscosity of the coating solutions at 25°C measured using an RFS fluid spectrometer manufactured by Rheometric Far East Co. was 1500, 220, 70, 40 and 20 mPa•s at shear rates of 0.1, 1, 10, 100 and 1000 1/sec., respectively.

The UF-purified SBR latex was obtained in the following manner.

The following SBR latex was diluted with distilled water ten times, and diluted and purified using a module for UF-purification, FSO3-FC-FUYO3A1 (Daisen Membrane System Co.) until the ion conductivity reached 1.5 mS/cm. At this time, the latex concentration was 40%.

(SBR Latex: Latex of -St(68)-Bu(29)-AA(3))
Average particle size: 0.1 μm, concentration: 45%, equilibrium moisture content at 25°C, 60% RH: 0.6% by weight, ion conductivity: 4.2 mS/cm (the ion conductivity was measured for a stock solution of the latex by use of a CM-30S conductivity meter manufactured by Towa Denpa Kogyo Co., pH: 8.2

〈Preparation of Coating Solution of Emulsion Surface Intermediate Layer〉

(Coating Solution for Intermediate Layer)

To 772 g of a 10 wt% aqueous solution of polyvinyl alcohol (PVA-205, manufactured by Kuraray Co., Ltd.) and 226 g of a 27.5% solution of methyl methacrylate/styrene/2-ethylhexyl acrylate/hydroxyethyl methacrylate/acrylic acid copolymer (copolymerization weight ratio: 59/9/26/5/1) latex, 2 ml of a 5 wt% aqueous solution of Aerosol OT (manufactured by American Cyanamide), 4 g of benzyl alcohol, 1 g of 2,2,4-trimethyl-1,3-pentanediol monoisobutylate and 10 mg of benzoisothiazolinone to prepare a coating solution for an intermediate layer, and the solution was supplied to a coating die so as to give 5 ml/m$^2$.

The viscosity of the coating solution measured with a B type viscometer (No. 1 rotor was used) at 40°C was 21 mPa•s.

〈Preparation of Coating Solution for First Emulsion Surface Protection Layer〉

(Coating Solution No. 1 for First Protection Layer)

Inert gelatin (80 g) was dissolved in water, and 138 ml of a 10% solution of phthalic acid in methanol, 28 ml of 1 N sulfuric acid, 5 ml of a 5 wt% aqueous solution of Aerosol OT (manufactured by American Cyanamide) and 1 g of phenoxyethanol were added thereto. Then, water was added thereto to bring the total volume to 1000 ml, thus preparing a coating solution, which was supplied to a coating die so as to give 10 ml/m$^2$.

The viscosity of the coating solution measured with a B type viscometer (No. 1 rotor was used) at 40°C was 17 mPa • s.

〈Preparation of Coating Solution for Second Emulsion Surface Protection Layer〉

(Coating Solution for Second Protection Layer)

Inert gelatin (100 g) was dissolved in water, and 20 ml of a 5% solution of N-perfluorooctylsulfonyl-N-propylalanine potassium salt, 16 ml of a 5 wt% aqueous solution of Aerosol OT (manufactured by American Cyanamide), 25 g of fine polymethyl methacrylate particles (average particle size: 4.0 μm), 44 ml of 1 N sulfuric acid and 10 mg of benzoisothiazolinone were added thereto. Then, water was added to bring the total amount to 1555 g, and 445 ml of an aqueous solution containing 4 wt% chrome alum and 0.67 wt% phthalic acid were mixed therewith with a static mixer just before coating, thus preparing a coating solution for a surface protection layer, which was supplied to a coating die so as to give 10 ml/m$^2$.

The viscosity of the coating solution measured with a B type viscometer (No. 1 rotor was used) at 40°C was 9 mPa • s.

〈Preparation of Back Face Coating Solution〉

(Preparation of Dispersion of Fine Solid Particles of Base Precursor〉

A base precursor compound (64 g) and 10 g of a surfactant, Demol N manufactured by Kao Corp. were mixed with 246 ml of distilled water, and the mixed solution was subjected to beads dispersion using a sand mill (a 1/4 gallon sand grinder mill, manufactured by Imex Co.) to obtain a dispersion of fine solid particles of the base precursor having an average particle size of 0.2 μm.

(Preparation of Dispersion of Fine Solid Particles of Dye)

A cyanine dye compound (9.6 g) and 5.8 g of sodium p-alkylbenzenesulfonate were mixed with 305 ml of distilled water, and the mixed solution was subjected to beads dispersion using a sand mill (a 1/4 gallon sand grinder mill, manufactured by Imex Co.) to obtain a dispersion of fine solid particles of the dye having an average particle size of 0.2 μm.

(Preparation of Coating Solution for Antihalation Layer)

Gelatin (17 g), 9.6 g of polyacrylamide, 70 g of the above-mentioned dispersion of fine solid particles of the base precursor, 56 g of the above-mentioned dispersion of fine solid particles of the dye, 1.5 g of fine polymethyl methacrylate particles (average particle size: 6.5 μm), 2.2 g of sodium polyethylenesulfonate, 0.2 g of a 1% aqueous solution of a coloring dye compound and 844 ml of water were mixed to prepare a coating solution for an antihalation layer.

(Preparation of Coating Solution For Protection Layer)

A vessel was kept hot at 40°C, and 50 g of gelatin, 0.2 g of sodium polyethylenesulfonate, 2.4 g of N,N'-ethylene-bis(vinylsulfoneacetamide), 1 g of sodium t-octylphenoxy-ethoxyethanesulfonate, 30 mg of benzoisothiazolinone, 32 g of $C_8F_{17}SO_3K$, 64 mg of $C_8F_{17}SO_2N(C_3H_7)$ $(CH_2CH_2O)_4(CH_2)_4$-$SO_3Na$ and 950 ml of $H_2O$ were mixed therein to prepare a coating solution for a protection layer.

Surfactant 1

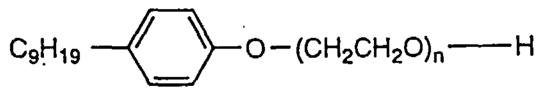

n = about 8.5

Spectral Sensitizing Dye 1

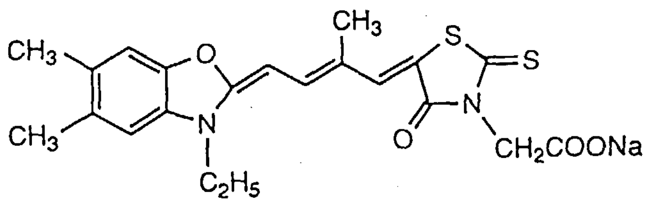

Tellurium Compound

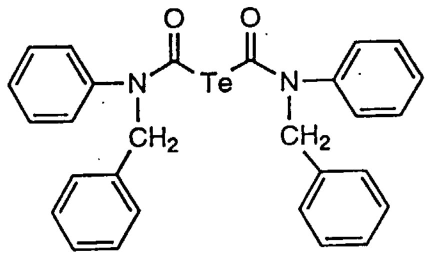

Base Precursor Compound

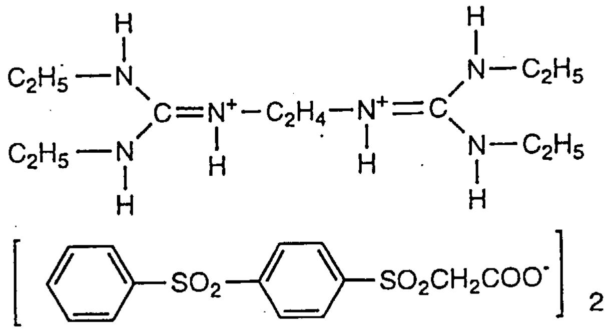

Cyanine Dye Compound

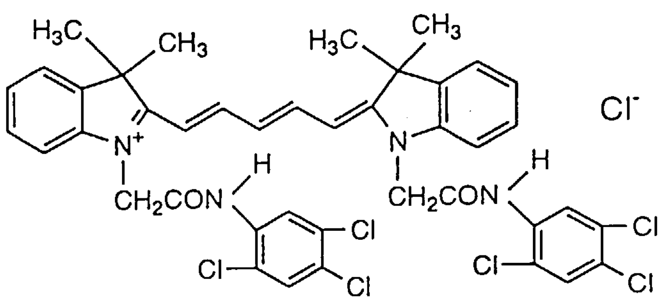

Coloring Dye Compound

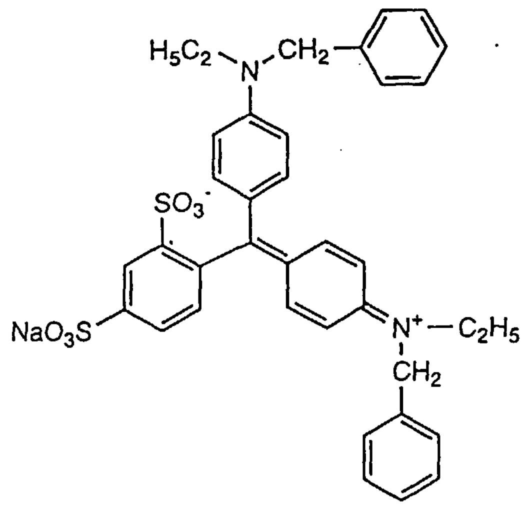

⟨Preparation of Photothermographic Light-sensitive Materials A to N⟩

The above-mentioned undercoated support was simultaneously coated with the coating solution for the antihalation layer so as to give an amount of solid matter of the fine solid particle dye coated of 0.04 $g/m^2$ and with the coating solution for the protection layer so as to give an amount of gelatin coated of 1 $g/m^2$ in multiple layers, followed by drying to prepare an antihalation back layer. Then, the emulsion layer, the intermediate layer, the first protection layer and the second protection layer were simultaneously coated in the slid bead coating system from the undercoat face opposite to the back face in this order in multiple layers to prepare a sample of the photothermographic light-sensitive material. After coating of the back face, the emulsion surface was coated without being wound.

The coating was carried out at a speed of 160 m/min., and the clearance between the tip of the coating die and the support was 0.18 mm. The pressure in a vacuum chamber was set at a pressure 392 Pa lower than atmospheric pressure. In a subsequent chilling zone, air having a dry-bulb temperature of 18°C and a wet-bulb temperature of 12°C was blown at an average air speed of 7 m/sec. for 30 seconds to cool the coating solutions. Thereafter, in a helical floating type drying zone, drying air having a dry-bulb temperature of 30°C and a wet-bulb temperature of 18°C was blown at a blowing air speed from a hole of 20 m/sec. for 20 seconds to evaporate the solvents in the coating solutions.

The photothermographic light-sensitive materials A to N prepared were evaluated by the following methods.

⟨Evaluation of Photographic Characteristics⟩

The photothermographic light-sensitive materials A to N were exposed to laser light of a Kr laser photographic sensitometer (maximum output: 500 mW) of 647 nm at an angle of 7 degrees to a normal, followed by processing (development) at 120°C for 15 seconds. The resulting images were evaluated with a densitometer. Results of the measurement were evaluated by Dmin and sensitivity (the reciprocal of a ratio of an exposure giving a density 1.0 higher than Dmin). As to the sensitivity, the sensitivity of light-sensitive material A was taken as 100. Higher value of sensitivity and lower Dmin value show better photographic characteristics.

⟨Evaluation of Light Transmission Properties⟩

The photothermographic light-sensitive materials A to N were exposed to laser light of a Kr laser photographic sensitometer (maximum output: 500 mW) of 647 nm at an angle of 7 degrees to a normal, followed by processing (development) at 120°C for 15 seconds. The samples were held to the monochromatic light of 660 nm, and functionally evaluated for the transparency.

◎: Completely transparent level
○ : Slightly clouded, but therein no problem in image reading.
△: Clouded, and a problem arises with regard to image reading.
X: Completely clouded level

⟨Evaluation of Forced Aging Keeping Quality⟩

The photothermographic light-sensitive materials A to N were cut to a size of 30.5 cm × 25.4 cm, and corners were rounded to round corners having an inside diameter of 0.5 cm. They were allowed to stand under the conditions of 25°C and 50% RH for 1 day. Ten sheets of each light-sensitive material were sealed in a bag made of a moisture-proof material, and further placed in a fancy box of 35.1 cm × 26.9 cm × 3.0 cm, followed by aging at 50°C for 5 days (forced aging). These samples and samples for comparison aged in the same manner as described above with the exception that the samples were kept at a temperature of 4°C, were subjected to the same processing as in the evaluation of photographic characteristics, and the density of fog portions was measured. The aging keeping quality was evaluated as the fog increasing rate.

Fog increasing rate = [{(Fog of forced aging sample) - (Fog of sample for comparison)}/{(Maximum density of sample for comparison) - (Density of support)}] × 100

Lower fog increasing rate shows better aging keeping quality.

⟨Evaluation of Light-irradiated Image Keeping Quality⟩

The light-sensitive materials exposed and developed in the same manner as with the evaluation of photographic characteristics were stuck on the inside of a glass window exposed to the direct rays of the sun, and allowed to stand for 1 month. Then, the images were visually evaluated according to the following standard:

◎ : Almost no changes
○ : Slight changes in color tone, but there is no problem.
△: Discolored in image areas, but practically allowable
X: Discolored in Dmin to increase the density, and unallowable

Results of evaluation for the above four items are shown in Table 3.

TABLE 3

| Light-Sensitive Material | Results of Photographic Characteristics | | Light Transmission Properties | Forced Aging Fog Increasing Rate | Light-Irradiation Image Keeping Quality |
|---|---|---|---|---|---|
| | Dmin | Sensitivity | | | |
| A (Invention) | 100 | 100 | ○ | 5 | ◎ |
| B (Invention) | 99 | 99 | ◎ | 10 | ◎ |
| C (Comparison) | 99 | 98 | ◎ | 238 | ◎ |
| D (Invention) | 100 | 102 | ○ | 2 | ○ |
| E (Comparison) | 102 | 102 | △ | 1 | △ |
| F (Invention) | 102 | 100 | ○ | 5 | ○ |
| G (Invention) | 103 | 100 | ○ | 10 | ○ |
| H (Invention) | 105 | 101 | ○ | 12 | ○ |
| I (Comparison) | 129 | 101 | ○ | 55 | △ |
| J (Comparison) | 100 | 100 | △ | 38 | △ |
| K (Comparison) | 100 | 99 | X | 6 | ○ |

TABLE 3 (continued)

| Light-Sensitive Material | Results of Photographic Characteristics | | Light Transmission Properties | Forced Aging Fog Increasing Rate | Light-Irradiation Image Keeping Quality |
|---|---|---|---|---|---|
| | Dmin | Sensitivity | | | |
| L (Comparison) | 105 | 100 | △ | 6 | ○ |
| M (Invention) | 104 | 99 | ○ | 9 | ○ |
| N (Invention) | 108 | 98 | ○ | 9 | ○ |

This shows that the photothermographic light-sensitive materials in which the keeping quality is compatible with the light transmission properties can be obtained by the present invention.

EXAMPLE 2

Light-sensitive materials A' to K' were prepared in the same manner as in Example 1 with the exception that the dispersions of the fatty acid silver salts were changed to ones prepared by the following methods.

〈Preparation of Fatty Acid Silver Salt A' (Invention)〉

Behenic acid (trade name: Edenor C22-85R) (87.6 g) manufactured by Henckel Co., 423 ml of distilled water, 49.2 ml of a 5 N aqueous solution of NaOH and 120 ml of tert-butanol were mixed, and stirred at 75°C for 1 hour to conduct the reaction, thereby obtaining a sodium behenate solution. Separately, 206.2 ml of an aqueous solution containing 40.4 g of silver nitrate (pH 4.0) was prepared, and the temperature thereof was kept at 10°C. A reaction vessel in which 635 ml of distilled water and 30 ml of tert-butanol were placed was kept at a temperature of 30°C, and the sodium behenate solution previously prepared and the aqueous solution of silver nitrate were wholly added thereto at a constant flow rate for 62 minutes and 10 seconds and for 60 minutes, respectively. At this time, only the aqueous solution of silver nitrate was added for 7 minutes and 20 seconds after the start of addition of the aqueous solution of silver nitrate. Thereafter, addition of the the sodium behenate solution was started, and only the sodium behenate solution was added for 9 minute and 30 seconds after addition of the aqueous solution of silver nitrate was completed. At this time, the temperature in the reaction vessel was adjusted to 30°C, and the temperature of the outside was controlled so that the liquid temperature was not elevated. Further, a pipe of an addition system of the sodium behenate solution was lagged with steamed jacket, and the opening of a valve for steam was controlled so that the liquid temperature at an outlet of a tip of an addition nozzle became 75°C. Further, a pipe of an addition system of the aqueous solution of silver nitrate was lagged by circulating cool water in the outer space of a double pipe. A position of adding the sodium behenate solution and a position of adding the aqueous solution of silver nitrate are arranged symmetrically centered on a stirring shaft, and at such a height that they do not come into contact with the reaction solution (refer to Figs. 1 and 3).

After addition of the sodium behenate solution was completed, the solution was allowed to stand with stirring for 20 minutes at a temperature left as it was, and then, the temperature was lowered to 25°C. Then, solid matter was filtered by suction filtration, and washed with water until a filtrate showed a conductivity of 30 μS/cm. Thus, fatty acid silver salt A' was obtained. The resulting solid matter was not dried and stored as a wet cake.

The shape of the resulting silver behenate particles was evaluated taking electron photomicrographs. As a result, the silver behenate particles were crystals in a scale shape having an average equivalent-sphere diameter of 0.52 μm, an average long side/short side of 1.5, an average aspect ratio of 5.1, an average particle thickness of 0.14 μm and a coefficient of variation of equivalent-sphere diameters of 15%.

〈Preparation of Fatty Acid Silver Salts B' to G'〉

Fatty acid silver salts B' to G' were prepared in the same manner as in fatty acid silver salt A' with the exception that the temperature of the sodium behenate solution, the temperature in the reaction vessel, the position of the addition nozzle and the height of the addition nozzle were changed as shown in Table 4 (refer to Figs. 1 to 4).

〈Preparation of Fatty Acid Silver Salt H'〉

Fatty acid silver salt H' was prepared in the same manner as in fatty acid silver salt A' with the exception that

the addition path of the sodium behenate solution was not lagged. At this time, the temperature of the sodium behenate solution was lowered to 60°C at the tip of the addition nozzle.

When the temperature in the vessel for preparing the sodium behenate solution was set at 70°C, the sodium behenate solution was solidified in the pipe of the addition path, resulting in failure to be added.

〈Preparation of Fatty Acid Silver Salts I' and J'〉

Fatty acid silver salts I' and J' were prepared in the same manner as in fatty acid silver salt A' with the exception that a 0.1 mol/liter aqueous solution of the nitrate of Ca or Zn was added so as to give a concentration of 1 mol% per mol (in terms of each metal ion) of silver nitrate. The shape of the fatty acid silver salts I' and J' is shown in Table A.

TABLE A

| Particle | Equivalent-Sphere Diameter (μm) | Long Side/Short Side | A. R. | Particle Thickness (μm) | Coefficient of Variation |
|---|---|---|---|---|---|
| A' | 0.52 | 1.5 | 5.2 | 0.14 | 15 |
| B' | 0.54 | 2.2 | 5 | 0.14 | 20 |
| C' | 0.62 | 10.4 | 4.9 | 0.18 | 32 |
| D' | 0.65 | 5.5 | 4.6 | 0.21 | 35 |
| E' | 0.68 | 7.8 | 4.2 | 0.22 | 38 |
| F' | 0.62 | 1.3 | 5.4 | 0.17 | 28 |
| G' | 0.53 | 1.9 | 4.3 | 0.15 | 25 |
| H' | 0.68 | 1.3 | 5.6 | 0.18 | 35 |
| I' | 0.53 | 1.5 | 5.2 | 0.14 | 16 |
| J' | 0.53 | 1.6 | 5.1 | 0.14 | 17 |

〈Preparation of Fatty Acid Silver Salt K'〉

Behenic acid (trade name: Edenor C22-85R) (43.8 g) manufactured by Henckel Co., 730 ml of distilled water, 60 ml of tert-butanol, and 117 ml of a 1 N aqueous solution of NaOH were mixed, and stirred at 79°C for 1 hour to conduct the reaction. Then, 112.5 ml of an aqueous solution containing 19.2 g of silver nitrate (pH 4.2) was added for 10 minutes. The solution was allowed to stand for 5 minutes as such, and then, the temperature was lowered to 30°C. Then, solid matter was filtered by suction filtration, and washed with water until a filtrate showed a conductivity of 30 μS/cm. Thus, fatty acid silver salt K' was obtained. The resulting solid matter was not dried and stored as a wet cake.

The shape of the resulting silver behenate particles was evaluated taking electron photomicrographs. As a result, the silver behenate particles were needle-like crystals having an average particle long axis length of 3 μm and an average particle short axis length of 0.06 μm.

TABLE 4

| Fatty Acid Silver Salt (°C) | Addition Temp. of Sodium Behenate (°C) | Temp. in Reaction Vessel (°C) | Difference in Temp. | Nozzle Position | Nozzle Height | Lagging of Sodium Behenate Pipe | Addition of Metal Salt | Note |
|---|---|---|---|---|---|---|---|---|
| A' | 75 | 30 | 45 | Sym. | Upper than solution surface | Lagged | - | Simultaneous addition |
| B' | 75 | 40 | 35 | Sym. | ditto | ditto | - | ditto |
| C'(Comparison) | 75 | 60 | 15 | Sym. | ditto | ditto | - | ditto |
| D'(Comparison) | 70 | 45 | 25 | Sym. | ditto | ditto | - | ditto |
| E'(Comparison) | 70 | 50 | 20 | Sym. | ditto | ditto | - | ditto |
| F' | 75 | 30 | 45 | Sym. | In solution | ditto | - | ditto |
| G' | 75 | 30 | 45 | Adjacent | Upper than solution surface | ditto | - | ditto |
| H' | 60 | 30 | 30 | Sym. | ditto | Not lagged | - | ditto |
| I' | 75 | 30 | 45 | Sym. | ditto | Lagged | Ca | ditto |
| J' | 75 | 30 | 45 | Sym. | ditto | ditto | Zn | ditto |
| K'(Comparison) | - | - | - | - | - | - | - | * |

* : silver nitrate was added to a solution of behenic acid and NaOH.

Fatty acid silver salts A' to K' obtained above were dispersed in the following manner to prepare dispersions A' to K' of the fatty acid silver salts.

〈Preparation of Dispersions A' to K' of Fatty Acid Silver Salts〉

To a wet cake corresponding to 100 g of dried solid matter of each of fatty acid silver salts A' to K', 7.4 g of polyvinyl alcohol (PVA-205, manufactured by Kuraray Co., Ltd.) and water were added to make the total weight 385 g, and the resulting mixture was preliminarily dispersed with a homomixer.

Then, the original fluid preliminarily dispersed was treated three times with a dispersing device (trade name: Microfluidizer M-110S-EH, manufactured by Microfluidex International Corporation, using a G10Z interaction chamber), adjusting its pressure to 1750 $kg/cm^2$. Thus, each of dispersed products A' to K' of the fatty acid silver salts was obtained. The particle size of dispersed products A' to K' of the fatty acid silver salts thus obtained is shown in Table 5. For the cooling operation in dispersion, coiled heat exchangers were each mounted in front of and behind the interaction chamber, and the temperature of a refrigerant was controlled thereby to set the dispersing temperature to a desired temperature.

〈Evaluation of Forced Aging Keeping Quality〉

The photothermographic light-sensitive materials A' to K' were cut to a size of 30.5 cm × 25.4 cm, and corners were rounded to round corners having an inside diameter of 0.5 cm. They were allowed to stand under the conditions of 25°C and 50% RH for 1 day. Ten sheets of each light-sensitive material were sealed in a bag made of a moisture-proof material, and further placed in a fancy box of 35.1 cm × 26.9 cm × 3.0 cm, followed by aging at 50°C for 5 days (forced aging). These samples and samples for comparison aged in the same manner as described above with the exception that the samples were kept at a temperature of 4°C, were subjected to the same processing as in the evaluation of photographic characteristics, and the density of fog portions was measured. The aging keeping quality was evaluated as the fog increasing rate.

Results of evaluation for the above three items are shown in Table 5. This shows that good Dmin can be obtained without deterioration of sensitivity, and that the aging keeping quality can be improved, by the present invention. Further, light-sensitive materials I' and J' exhibit a further improved effect of the image keeping quality, which shows the usefulness of the present invention.

**TABLE 5**

| Dispersion or Light-Sensitive Material | Volume Weighted Average Diameter of Dispersion (μm) | Coefficient of Variation (%) | Results of Photographic Characteristics | | Forced Aging Fog Increasing Rate | Results of Image Keeping Quality |
|---|---|---|---|---|---|---|
| | | | Dmin | Sensitivity | | |
| A' | 0.52 | 15 | 100 | 100 | 1.0 | ○ |
| B' | 0.54 | 20 | 101 | 100 | 1.2 | ○ |
| C' (Comparison) | 0.62 | 32 | 150 | 97 | 4.5 | × |
| D' (Comparison) | 0.65 | 35 | 140 | 96 | 5.2 | × |
| E' (Comparison) | 0.68 | 38 | 165 | 92 | 6.3 | × |
| F' | 0.62 | 28 | 125 | 94 | 3.8 | △ |
| G' | 0.53 | 25 | 120 | 93 | 3.6 | △ |
| H' | 0.68 | 35 | 115 | 95 | 3.2 | △ |
| I' | 0.53 | 16 | 85 | 105 | 0.8 | ◎ |
| J' | 0.53 | 17 | 75 | 102 | 0.7 | ◎ |
| K' (Comparison) | 0.75 | 38 | 195 | 89 | 9.3 | × |

EXAMPLE 3

Fatty acid silver salt T' was prepared in the same manner as in the fatty acid silver salt A' prepared in Example 2, and then, dispersion T' of the fatty acid silver salt was obtained as follows.

〈Preparation of Dispersion T' of Fatty Acid Silver Salt〉

To a wet cake corresponding to 100 g of dried solid matter of fatty acid silver salt T', 7.4 g of polyvinyl alcohol (PVA-205, manufactured by Kuraray Co., Ltd.) and water were added to make the total weight 385 g, and the resulting mixture was preliminarily dispersed with a homomixer.
Then, this solution was placed in a 1/4 gallon vessel containing 912 g of zirconia beads, and dispersed with a sand grinder mill (manufactured by Imex Co.) at 1500 rpm for 5 hours while keeping the internal temperature at 20°C.
Dispersion T' of the fatty acid silver salt thus obtained was applied in the same manner as in Example 1 to obtain photothermographic light-sensitive material T'.

〈Evaluation of Surface State〉

Using photothermographic light-sensitive material T' and photothermographic light-sensitive material A' used in Example 2, exposure and development were conducted in the same manner as in the evaluation of photographic characteristics, and the 10 cm density thereof was measured in a coating width direction by every 1 mm. The value obtained by multiplying the standard deviation of the density by 100 is shown in Table 6. The lower this value is, the better the surface state is. Table 6 shows that photothermographic light-sensitive material A' is small in density variation in the coating width direction, and suitable for practical use.
For the Dmin, the sensitivity, the fog increasing rate and the image keeping quality, light-sensitive materials A' and T' showed approximately similar results.

TABLE 6

| Light-Sensitive Material | Density Variation in Coating Width Direction (standard deviation × 100) |
|---|---|
| A' | 8 |
| T' | 35 |

It is seen that in the present invention, the photothermographic light-sensitive materials low in fog and excellent in aging keeping quality and image keeping quality are obtained.

## Claims

1. An aqueous dispersion of fatty acid silver salt particles which are dispersed in an aqueous solvent, wherein said fatty acid silver salt particles are ones formed by simultaneously adding into a reaction vessel (a) a silver ion-containing aqueous solution or a silver ion-containing water-organic solvent mixed solution and (b) an aqueous solution of an alkali metal salt of a fatty acid, a solution thereof in a water-organic solvent mixed solvent or a solution thereof in an organic solvent, in an amount of at least 10% of the total amount of silver to be added, and the fatty acid silver salt particles formed satisfies all the following characteristics:

   (1) The average equivalent-sphere diameter of the fatty acid silver salt particles is from 0.1 μm to 0.8 μm;
   (2) The average ratio of long sides/short sides in main planes of the fatty acid silver salt particles is from 1 to 4;
   (3) The average aspect ratio of the particle is from 2 to 30; and
   (4) The average thickness of the particles is from 0.01 μm to 0.20 μm.

2. The aqueous dispersion of the fatty acid silver salt particles as in claim 1, wherein said fatty acid silver salt particle is formed by simultaneously adding the components (a) and (b) in an amount of at least 50% of the total amount of silver to be added.

**3.** The aqueous dispersion of the fatty acid silver salt particles as in claim 1, wherein the coefficient of variation of the size of the fatty acid silver salt particles formed is 20% or less.

**4.** The aqueous dispersion of the fatty acid silver salt particles as in claim 1, wherein the maximum concentration of the fatty acid silver salt particles during particle formation is 0.12 mol/liter to 0.5 mol/liter.

**5.** A method for producing an aqueous dispersion of fatty acid silver salt particles which are dispersed in an aqueous solvent, which comprises simultaneously adding into a reaction vessel (a) a silver ion-containing aqueous solution or a silver ion-containing water-organic solvent mixed solution and (b) an aqueous solution of an alkali metal salt of a fatty acid, a solution thereof in a water-organic solvent mixed solvent or a solution thereof in an organic solvent, in an amount of at least 10% of the total amount of silver to be added, the fatty acid silver salt particles formed satisfying all the following characteristics:

(1) The average equivalent-sphere diameter of the fatty acid silver salt particles is from 0.1 μm to 0.8 μm;
(2) The average ratio of long sides/short sides in main planes of the fatty acid silver salt particles is from 1 to 4;
(3) The average aspect ratio of the particle is from 2 to 30; and
(4) The average thickness of the particles is from 0.01 μm to 0.20 μm.

**6.** The method as in claim 5, wherein at least 50% of the total amount of silver to be added of the components (a) and (b) is simultaneously added.

**7.** The method as in claim 5, wherein the coefficient of variation of the size of the fatty acid silver salt particles formed is 20% or less.

**8.** The method as in claim 5, wherein the maximum concentration of the fatty acid silver salt particles during particle formation is 0.12 mol/liter to 0.5 mol/liter.

**9.** The method as in claim 5, wherein an aqueous solution of a tertiary alcohol containing said fatty acid alkali metal salt is added to the solution in said reaction vessel, and the temperature difference between the solution in said reaction vessel and the aqueous solution of the tertiary alcohol containing said fatty acid alkali metal salt is from 30°C to 85°C.

**10.** The method as in claim 9, wherein a tip of a nozzle for adding the aqueous solution of the tertiary alcohol containing said fatty acid alkali metal salt to the solution in the reaction vessel does not come into contact with the solution in said reaction vessel.

**11.** The method as in claim 9, wherein a path of from a vessel in which the aqueous solution of the tertiary alcohol containing said fatty acid alkali metal salt is prepared to the nozzle for adding the aqueous solution to the solution in the reaction vessel is kept hot so as to maintain the aqueous solution of the tertiary alcohol containing said fatty acid alkali metal salt in the transfer path at a constant temperature of 65°C to 85°C.

**12.** The method as in claim 5, which further comprises a desalting process after formation of the fatty acid silver salt.

**13.** A method for redispersing the fatty acid silver salt particles of the aqueous dispersion according to claim 1 in an aqueous solvent, which comprises crudely dispersing the fatty acid silver salt particles in the aqueous solvent to prepare a crude dispersion, and then, converting the crude dispersion to a high-pressure high-speed stream, followed by a reduction in pressure.

**14.** A photothermographic light-sensitive material comprising a support having thereon a light-sensitive layer containing at least a fatty acid silver salt, a reducing agent for silver ions, a binder and a light-sensitive silver halide, wherein said light-sensitive layer is formed by applying a coating solution in which 30% by weight or more of a solvent is water, and drying it, and the coating solution for said light-sensitive layer contains a polymer dispersed in a latex form as a main binder, and the fatty acid silver salt particles of the aqueous dispersion according to claim 1 as a dispersion.

**15.** A method for producing a photothermographic light-sensitive material by applying a coating solution for a light-sensitive layer containing a fatty acid silver salt, a reducing agent for silver ions, a binder and a light-sensitive silver halide onto a support, and drying it, wherein the coating solution for said light-sensitive layer contains 30% by weight

or more of water as a solvent, a polymer dispersed in a latex form as a main binder, and the fatty acid silver salt particles dispersed by the redispersing method according to claim 13 as a dispersion.

**16.** The method as in claim 15, wherein at least one metal ion selected from the group consisting of Ca, Mg, Ce, Al, Zn and Ba is added in any of steps of from a step for producing said fatty acid silver salt to a step for applying the coating solution for the light-sensitive image forming layer containing said fatty acid silver salt obtained by said producing method.

Fig. 1

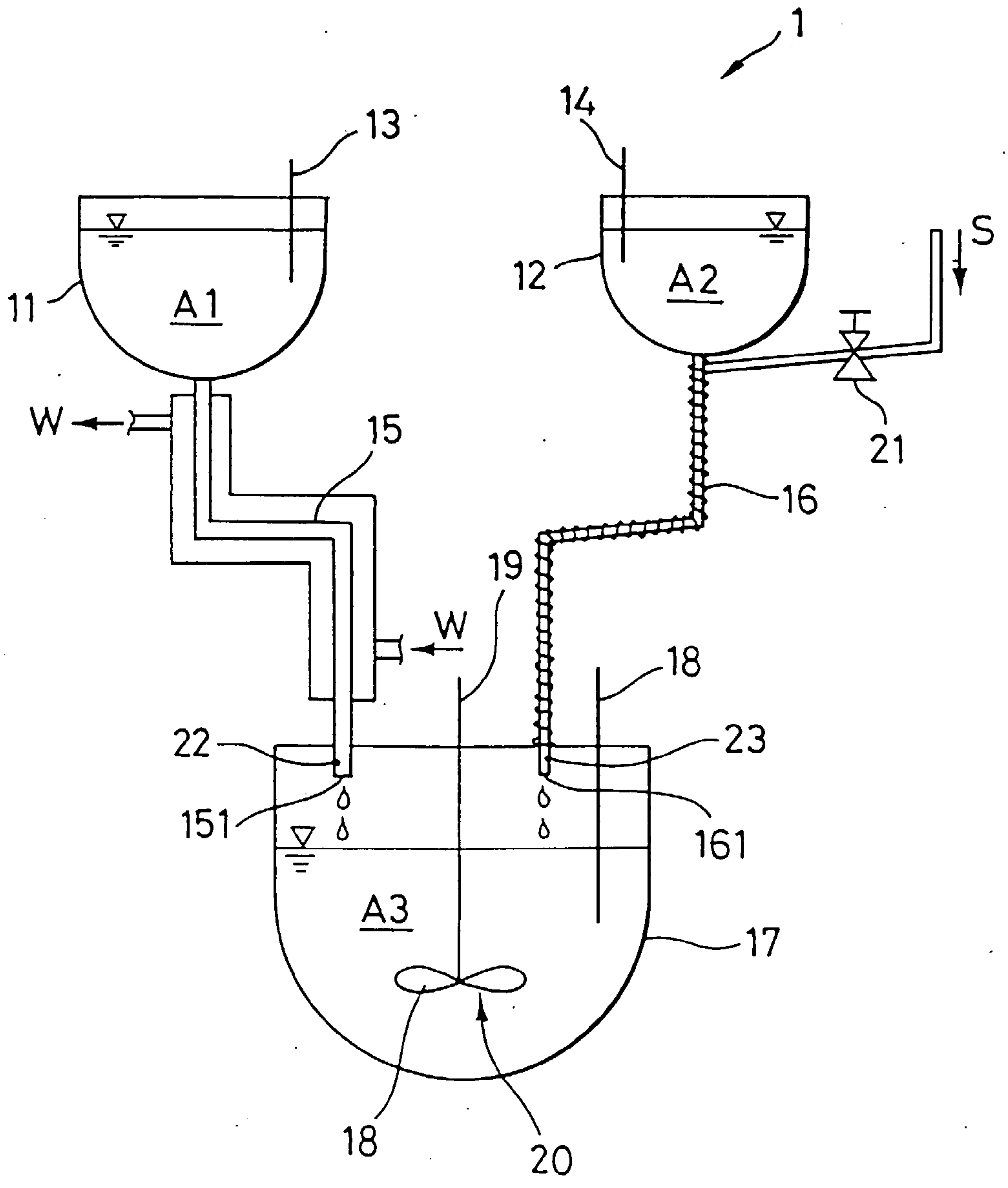

Fig. 2

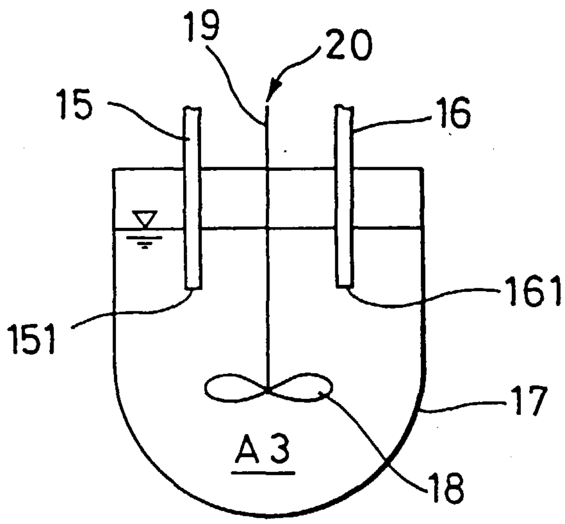


Fig. 3

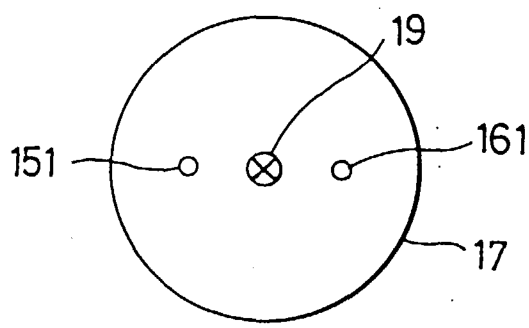


Fig. 4

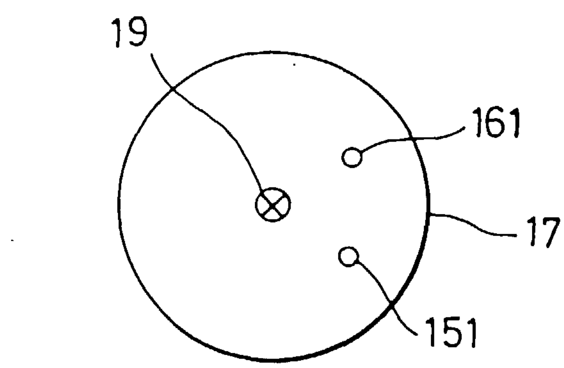

European Patent Office

# EUROPEAN SEARCH REPORT

Application Number
EP 99 11 0902

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,A | EP 0 754 969 A (AGFA GEVAERT NV) 22 January 1997 (1997-01-22) * column 20, line 48 - line 54 * * comparative example 1; figure 2 * | 1 | G03C1/498 C07C51/41 |
| A | US 4 157 289 A (IKENOUE SHINPEI ET AL) 5 June 1979 (1979-06-05) * column 2, line 35 - line 46; figure 2 * | 1,5 | |
| A | US 4 273 723 A (HAYASHI YOSHIO ET AL) 16 June 1981 (1981-06-16) * column 2, line 50 - line 68; claims 1-10; figure 1 * | 1,5 | |
| A | GB 1 378 734 A (FUJI PHOTO FILM CO LTD) 27 December 1974 (1974-12-27) * page 3, line 23 - line 42 * | 1,5 | |
| A | DATABASE WPI Section Ch, Week 9632 Derwent Publications Ltd., London, GB; Class G06, AN 96-312982 XP002116080 & JP 08 137044 A (CANON KK), 31 May 1996 (1996-05-31) * abstract * | 13 | TECHNICAL FIELDS SEARCHED (Int.Cl.6) G03C C07C |
| A | DATABASE WPI Section Ch, Week 8225 Derwent Publications Ltd., London, GB; Class E12, AN 82-51289E XP002116089 & JP 57 077642 A (KINDAI KAGAKU KK), 15 May 1982 (1982-05-15) * abstract * | 13,16 | |

The present search report has been drawn up for all claims

| Place of search | Date of completion of the search | Examiner |
|---|---|---|
| THE HAGUE | 23 September 1999 | Bolger, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 99 11 0902

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-1999

| Patent document cited in search report | Publication date | Patent family member(s) | Publication date |
|---|---|---|---|
| EP 0754969 A | 22-01-1997 | WO 9704355 A | 06-02-1997 |
| | | WO 9704356 A | 06-02-1997 |
| | | WO 9704357 A | 06-02-1997 |
| | | EP 0839337 A | 06-05-1998 |
| | | EP 0840906 A | 13-05-1998 |
| | | EP 0839338 A | 06-05-1998 |
| | | JP 9127643 A | 16-05-1997 |
| | | US 5891616 A | 06-04-1999 |
| US 4157289 A | 05-06-1979 | JP 1130652 C | 17-01-1983 |
| | | JP 53137096 A | 30-11-1978 |
| | | JP 57021488 B | 07-05-1982 |
| | | DE 2819855 A | 09-11-1978 |
| | | GB 1580317 A | 03-12-1980 |
| US 4273723 A | 16-06-1981 | JP 1290441 C | 29-11-1985 |
| | | JP 55040607 A | 22-03-1980 |
| | | JP 60014010 B | 11-04-1985 |
| | | BE 878772 A | 13-03-1980 |
| | | CA 1118442 A | 16-02-1982 |
| | | DE 2936281 A | 20-03-1980 |
| | | FR 2436129 A | 11-04-1980 |
| | | GB 2038314 A,B | 23-07-1980 |
| GB 1378734 A | 27-12-1974 | JP 49001511 A | 08-01-1974 |
| | | JP 49011814 A | 01-02-1974 |
| | | DE 2322096 A | 22-11-1973 |
| | | FR 2183143 A | 14-12-1973 |
| | | US 3887597 A | 03-06-1975 |
| JP 8137044 A | 31-05-1996 | NONE | |
| JP 57077642 A | 15-05-1982 | JP 1769573 C | 30-06-1993 |
| | | JP 63057414 B | 11-11-1988 |

EPO FORM P0459

For more details about this annex : see Official Journal of the European Patent Office, No. 12/82